# EUROPEAN PATENT APPLICATION

(11) **EP 4 374 834 A1**
(43) Date of publication of application: **29.05.2024**
(21) Application number: 22845407.0
(22) Date of filing: 21.07.2022
(51) Int. Cl.: A61F 9/00

(54) **OPHTHALMIC INJECTION ASSEMBLY AND INJECTION DEVICE, AND USE METHOD**

(30) Priority: 22.07.2021 CN 202110830969
(71) Applicant: Chengdu Origen Biotechnology Co., Ltd., Chengdu, Sichuan 610037 (CN)
(72) Inventor: KE, Xiao, Chengdu, Sichuan 610037 (CN); ZHENG, Qiang, Chengdu, Sichuan 610037 (CN); JIANG, Hao, Chengdu, Sichuan 610037 (CN); LONG, Yang, Chengdu, Sichuan 610037 (CN); QIN, Yingfei, Chengdu, Sichuan 610037 (CN)
(74) Representative: Botti & Ferrari S.p.A.
(86) International application number: PCT/CN2022/107104
(87) International publication number: WO 2023/001244

(57) **Abstract**

The present invention relates to the field of ophthalmic therapies, specifically to ocular injection assemblies, injection devices and methods of use. The ocular injection assembly comprises a sleeve and a needle. The needle is capable of being set within the sleeve. A clamping port is provided at the end of the sleeve. A tip portion of the needle can pass through the clamping port during use. When using the ocular injection assembly, the clamping port of the sleeve is contacted with and pressed against the ocular tissue at the injection site to cause the ocular tissue at the injection site to protrude into the sleeve, which can significantly improve the success rate of the injection.

## Description

### Technical field

The present invention relates to the field of ophthalmic therapeutics and specifically to ocular injection assemblies, injection devices and methods of use.

### Background art

Eye is a very complex organ. As a visual organ, an eye consists of three parts: eyeball, visual pathway and accessory structures, with the eyeball and the visual pathway accomplishing the visual function. Eyeball is nearly spherical and consists of an eyeball wall and contents therein, which has a very complex structure. The wall of the eyeball contains three layers, the outer layer is the fibrous tunic, the middle layer is the uvea, and the inner layer is the retina. Fibrous tunic is mainly composed of fibrous tissue and forms the outer membrane of the eyeball, mainly cornea and sclera, of which the sclera accounted for 5/6. Sclera has tough texture composed of densely interwoven fibers. The sclera has different thickness in different parts of the eyeball and is individually specific among children, adults, and the elderly. The arrangement of the collagen fiber bundles within the sclera varies in different parts of the eye. The uvea situated between the sclera and the retina contains three contiguous parts from anterior to posterior: the iris, the ciliary body, and the choroid. The choroid consists of fibrous tissue, small blood vessels, and capillaries, and is a highly vascularized and pigmented tissue containing a meshwork of large branching small arterioles and arterioles as well as venous channels, the thickness of which varies with changes in vascular filling and decreases with age.

The eye, as an exposed organ, is susceptible to a variety of damages such as conjunctival or corneal injury caused by pathogens. The conjunctiva is a thin, transparent mucous membrane rich in blood vessels that covers the inner surface of the eyelid and the front of the eyeball, helping to prevent damage to the eye from foreign bodies and infections. However, the conjunctiva itself is not only very sensitive and prone to irritation by chemicals or allergic substances, or infected by viruses or bacteria to cause conjunctivitis, but also liable to guide a large amount of medication (>60%) into the system circulation through its rich blood vessels, causing tissue toxicity and other complications.

Currently, fundus diseases are one of the leading causes of irreparable visual impairment or loss. These fundus diseases include neovascular age-related macular degeneration, diabetic retinopathy, diabetic macular edema, central retinal vein occlusion and branch retinal vein occlusion. Ocular drug delivery devices play a critical role in the treatment of ocular diseases because tissue barriers (e.g., cornea, conjunctiva, blood-aqueous barrier, and blood-retinal barrier) limit drug delivery to the fundus. Conventional delivery methods such as ocular surface administration and intravitreal injection (IVT) are convenient but difficult to deliver drugs to the fundus efficiently and safely. Moreover, ocular injections that are performed in an incorrect position or at an incorrect angle may lead to complications, such as intraocular hemorrhage, or retinal damage, resulting in cataracts, retinal detachment, and so on. Infection of other tissues in the eye may be caused if leakage of the drug occurs.

Delivery of drugs to the eye has always been a challenge due to the unique structure of the eye. Delivery to the suprachoroidal space is particularly difficult due to the structure of the suprachoroidal space. The suprachoroidal space exists without a significant gap in the absence of fluid and/or tissue separation and is a potential space between the sclera and choroid. When fluid or other material accumulates between the choroid and the sclera, the suprachoroidal space may become visible in that region. Thus, by delivering, injecting and/or infusing a drug concoction into the suprachoroidal space, a fluid accumulation is intentionally created to further create and/or expand the separation of the choroid from the sclera, thereby creating a suprachoroidal space. Localized choroidal hemorrhage and retinal damage may occur during injection of drugs administered into the suprachoroidal space. Complications such as endophthalmitis, scleral dilatation, wound abscesses, and occasionally high intraocular pressure and cataracts may also occur as a result of injection.

In the prior art, devices for injecting drugs into the choroidal cavity are disclosed, but all of them produce side effects of damage to the eye. For example, CN 11 21 65923A discloses a device for injecting substance into the interlayer of a bodily tissue or an organ, wherein the injection needle comprises two layers of needles including an outer layer of a short 27G stainless steel needle, and a blunt separating needle inside thereof. First, the 27G tunneling needle obliquely pierces into the sclera from the corneal rim, the direction of pierce being kept parallel to the surface of the eyeball; the control button on the syringe is pressed to stretch out the separating needle to strip the tissue and form an intra-scleral channel connecting to the suprachoroidal space; and then the obtuse separating needle is withdrawn by the control knob on the syringe; and finally, the medicinal solution inside the syringe is injected into the suprachoroidal space to complete the injection through the passageway punched out by the tunneling needle. The device is more damaging to the eye, prone to cause bleeding, and the piercing angle is very critical and difficult to precisely control to accurately separate the channel to the suprachoroidal space, and prone to puncture into the vitreous body or puncture the conjunctiva. A therapeutic agent delivery device is disclosed in CN107223042B. In performing an ocular injection, the sclera is first incised behind the corneal limbus. A cannula with a curved opening is inserted between the sclera and the choroid and advanced to the suprachoroidal space, and then an injection needle is pushed into the subretina to complete the injection. However, this delivery device requires incision of the sclera and pushing the cannula through the eye wall, which is damaging and causes extensive scraping of the choroid and sclera. It is also difficult to control the depth of the needle, as it is easy to penetrate the retina.

In order to solve the above problems, there is a need to provide a simple and convenient device that can effectively deliver the drug to the target tissues in the eye and also solve the problem of injection-induced eye injuries.

### Summary of the invention

The present invention provides an ocular injection assembly which presses on the conjunctival tissue around the injection point through a clamping port to form a bulge facing the clamping port, which is capable of both effectively delivering a drug to a target tissue in the eye and solving the problem of injection-induced ocular injury.

In order to achieve the above purposes, the present invention comprises:
An ocular injection assembly comprising a sleeve and a needle, the needle being capable of being set within the sleeve, the sleeve having a clamping port at the end thereof, the tip portion of the needle being capable of passing through the gripping port. When the ocular injection assembly is used, the clamping port of the sleeve is contacted with and pressed against ocular tissues at the site of the injection, so as to cause the ocular tissues at the site of the injection to protrude into the sleeve.

Preferably, the clamping port has a minimum inner diameter of 1 mm to 3 mm. The needle is threaded into the injection point for injection, and the minimum inner diameter of the clamping port is the length of a line section passing through the injection point with both ends thereof inside the clamping port. The injection point is located in the middle of the line section. The experimental results showed that at an inner diameter of 1-3 mm, the area of ocular tissues inside the clamping port was moderate, and the ocular tissues were able to form a clear bump inside the clamping port, thereby facilitating the injection operation.

Preferably, the clamping port has an annular end face. The periphery of the clamping port forms an annular end surface capable of forming an annular face contact with the ocular tissue surrounding the injection site.

Preferably, the annular end face is planar and the needle is perpendicular to the annular end face. That is, the axial direction of the needle is perpendicular to the plane in which the annular end face is located.

Preferably, the clamping port is of round, oval or polygonal shape. Different shapes of the clamping port, such as round, oval, hexagonal, octagonal, square or irregular shape, can realize the pressure of the clamping port on the conjunctival tissue. When the clamping port is round, the annular end surface is circular, which is not only more conducive to avoid damage to the ocular tissues, but also more conducive to the recovery of the ocular tissues during the injection process.

Preferably, the end of the sleeve is a shrinking section, and the clamping port is located at the end of the shrinking section. The size of the cross-section of the shrinking section gradually increases in a direction away from the clamping port, which facilitates collecting and storing the refluxed liquid in the shrinking section in the event of reflux.

Preferably, the side wall of the sleeve is provided with an observation window made of transparent material, or the sleeve is a transparent material member. Providing an observation window or adopting a sleeve made of transparent material facilitates observing the return flow of the drug solution and quickly assessing whether the injection is successful.

Preferably, the observation window or the transparent sleeve is provided with a volume scale line set in the axial direction along the sleeve which enables real-time observation of the reflux of the medicinal fluid, and when the warning limit is exceeded, can conveniently remind the operator of the injection failure and thus stop the injection.

Preferably, the length of the edge surface of the needle tip portion is less than 1100 micrometers, preferably less than 900 micrometers, further preferably less than 700 micrometers, more preferably ≤ 550 micrometers, and most preferably 250-550 micrometers.

Preferably, when the needle is mated with the sleeve, the length of the needle tip portion outside the clamping port portion is 500-2000 micrometers, preferably 700-1350 micrometers, more preferably 700-1100 micrometers.

Preferably, in the case of a suprachoroidal space injection, the length of the tip portion of the needle outside the clamping portion is 500-1100 micrometers.

Preferably, the sleeve is provided with a canal and the needle is movably coupled to the canal. The needle is coupled to the sleeve via the canal, which facilitates the needle to maintain a relative position to the clamping port of the sleeve when piercing to reach the injection site.

Preferably, a needle hub is attached to the needle at an end opposite to the tip portion, and an adjusting assembly is provided between the sleeve and the needle hub for adjusting the size of the length of the tip portion of the needle outside the clamping port portion, facilitating the adjustment of the length of the tip of the needle outside the clamping port, and controlling the depth of the puncture, so as to be applicable to different patients, or to puncture different locations of the eye, such as applying to the eye wall tissue for patients with abnormal sclera thickness .

Preferably, the needle hub is coupled to the sleeve via the adjustment assembly. Setting the needle hub facilitates coupling with the syringe. Setting the adjustment assembly between the needle hub and the sleeve makes it less likely to be affected by other components such as the syringe when adjusting the length of the needle.

Preferably, the adjusting assembly comprises an externally threaded section provided on the needle hub, and an internally threaded section provided on a corresponding sleeve, the externally threaded section and internally threaded section being connectable to each other. The needle hub and the sleeve are rotatable relative to each other through the mating of the externally threaded section and the internally threaded section to realize the adjustment of the length of the needle.

Preferably, the needle hub comprises a guide tube provided with the externally threaded section. The guide tube is connected to the front end of the needle hub, and the guide tube set is connected to the needle for reinforcing the strength of the needle and avoiding bending or shaking of the needle during puncture process.

Preferably, the thread pitch is 50-200 microns, more preferably 50-150 microns.

The adjustment assembly may also be as follows.

A needle hub is connected to the rear end of the sleeve and is provided with an adjusting assembly comprising a telescopic rod assembly and a driving mechanism. The telescopic rod assembly comprises an outer tube and an inner tube, one end of the outer tube is connected to the rear end of the needle hub and the other end is sheathed on the inner tube, and the other end of the inner tube is connected to the rear end of the needle. The outer tube, the inner tube, and the needle are connected in turn. Alternatively, the rear end of the needle passes through the inner tube and the outer tube in turn and extends outside the outer tube. A driving mechanism is connected to the inner tube for driving the inner tube to move axially relative to the outer tube, and the inner tube is capable of driving the needle.

The rear end of the needle passing sequentially through the inner tube and the outer tube and outside the outer tube means that the needle is fixed to the inner tube and the rear end of the needle extends outside the inner tube toward the outer tube and to the other end of the outer tube.

Preferably, the needle is provided with a guide tube over the outer sleeve of the needle, the two ends of the needle are exposed from the guide tube, the front end of the needle hub is set outside the guide tube, and the guide tube and the needle hub are capable of sliding relative to each other. By setting the guide tube on the needle, the strength of the needle is strengthened, and the shaking and deformation of the needle during the puncturing of the ocular tissues are reduced.

Preferably, the driving mechanism includes a driving housing, a rack guide, a driving gear and an operating lever. The drive housing is connected to the outer tube at one end and slidingly connected to the inner tube at the other end. The rack guide is connected to the inner tube in an axial direction. The drive gear is hingedly connected to the drive housing and is engagedly connected to the rack guide. One end of the operating lever is connected to the drive gear, and the other end passes through the through-hole of the drive housing and the through-hole of the needle hub to the outside of the needle hub. By rotating the operating lever in the driving mechanism to rotate the driving gear, the driving gear moves with the rack guide, and the inner tube moves with the rack guide, thereby driving the needle to move in the axial direction for adjustment of the position of the needle.

The adjustment assembly may also be used in conjunction with the release assembly.

Preferably, a needle hub is connected to the rear end of the sleeve; a release assembly comprising a resilient member and a first stop member is provided between the sleeve and the needle hub; the sleeve is connected to the needle hub via the resilient member; one end of the first stop member is detachably connected to the sleeve and/or the other end of the first stop member is detachably connected to the needle hub. When the first stop member is connected to the sleeve and the needle hub, the resilient member is in stretching status and is capable of providing an axial tensile force to stretch the resilient member to increase the distance between the needle hub and the sleeve for inserting the first stop member into place to maintain the distance between the needle hub and the sleeve. At this point the resilient member is in the stretched state and the front end of the needle is retracted into the sleeve. During use, the clamping port is kept in contact with the ocular tissue, and after the first stop member is removed, the needle hub is moved forward so that the tip of the needle exceeds the clamping port and penetrates into the ocular tissue.

The resilient member may be a reset spring, a compressed gas container, or a container comprising a propellant; and the first stop member may be a tab, a slot, a ring, a slot, or a pawl.

Preferably, the adjustment assembly may be provided between the sleeve and the needle hub, the adjustment assembly comprising a telescopic rod assembly and a driving mechanism. The telescopic rod assembly comprises an outer tube and an inner tube, one end of the outer tube being connected to the front end of the needle hub, the other end being fit over the inner tube, and the other end of the inner tube being connected to the rear end of the needle. The outer tube, the inner tube and the needle are connected in turn, or the rear end of the needle passes through the inner tube and the outer tube in turn, and exceeds the outer tube. The driving mechanism is connected to the inner tube for driving the inner tube to move relative to the outer tube in an axial direction, and the inner tube is capable of driving the needle to move. In this embodiment, the structure of the adjusting assembly may be with the same as that in the previous embodiment, or the adjusting assembly may be provided outside of the needle hub and located in the middle of the resilient member.

Preferably, the driving mechanism comprises a driving housing, a rack guide, a driving gear and an operating lever; the driving housing is connected to the outer tube at one end and is slidingly connected to the inner tube at the other end; the rack guide is connected to the inner tube in the axial direction; the driving gear is hingedly attached to the driving housing and is meshing with the rack guide; one end of the operating lever is connected to the driving gear, and the other end passes through the through hole of the driving housing and the through hole of the needle hub to the outside of the needle hub. Turning the operating lever can rotate the drive gear. The operating lever and the drive gear are removably connected. When the operating lever is separated from the needle hub, the first stop member can be driven to be separated from the sleeve and/or the needle hub. By setting the operation lever outside the needle hub, the operation lever can be removed by pulling the operation lever out after adjusting the length of the needle using the operation lever to facilitate observation. Using the operation lever to drives the first stop member to separate from the sleeve and the needle hub facilitates one-handed operation by the operator.

Preferably, when the clamping port of the sleeve contacts and presses on the ocular tissue, the clamping port is sealed and the ocular tissue forms a bulge towards the inside of the sleeve.

Preferably, when the clamping port is sealed, the chamber within the sleeve forms a hermetically sealed chamber. In the event of reflux, the refluxed medicament is stowed in the sealed chamber, and when the clamping port is separated from the ocular tissues, it is difficult for the medicament to leak out of the sealed chamber, so as to avoid the medicament dispersing to the ocular tissues, which is advantageous for accurately determining the volume of the refluxed liquid and estimating the amount of liquid injected.

The present invention also provides an ocular injection device comprising a syringe, and an ocular injection assembly capable of being mounted on the syringe as described above.

Preferably, the syringe comprises a medication container storing the medication and fitting with a needle, and a pushrod being slidable within the medication container.

Preferably, a thrust assembly is provided between the end of the pushrod and the medication container for generating a constant thrust force on the pushrod.

The thrust assembly may be a spring-loaded ball mechanism, a spring-loaded pin, a cylinder, or a container containing propellant.

Preferably, the constant thrust generated by the thrust assembly has a threshold value less than 6N. The constant thrust generated by the thrust assembly means that when the thrust force applied to the thrust assembly is less than the threshold value, the thrust assembly cannot be pushed; and when the thrust force is greater than the threshold value, the thrust assembly can counteract the thrust force exceeding threshold value so that the thrust force is maintained at the threshold value. For example, when the threshold value is 5N, the thrust assembly cannot be pushed with a force of 4N, and when the thrust force is 7N, the thrust assembly will maintain a thrust force of 6N.

Preferably, the injection device further comprises a second stop member provided between the pushrod and the medication container for limiting the pushing stroke of the pushrod.

The second stop member may be a tab, slot, ring, slot or pawl.

Preferably, the injection device further comprises a needle protection cap cooperating with the needle.

The present invention also provides a method of using the ocular injection device described above which uses the ocular injection device to contact and press the clamping port of the sleeve against the ocular tissue at the injection site to form a bulge into the sleeve.

Preferably, after the clamping port is contacted and pressed against the ocular tissue at the injection site to form a bulge of the ocular tissue at the injection site into the sleeve, the tip portion of the needle is then made to puncture the ocular tissue at the injection site so that the distal end of the needle reaches the target ocular tissue at the injection site.

Preferably, while the clamping port contacts and presses the ocular tissue at the injection site to form a bulge of the ocular tissue at the injection site into the sleeve, the tip portion of the needle is made to puncture the ocular tissue at the injection site so that the distal end of the needle reaches the target ocular tissue at the injection site.

Preferably, the length of the tip portion of the needle that is exposed to the clamping port is adjusted using the adjustment assembly in the injection device described above according to the thickness of the ocular tissue at the injection site before the clamping port contacts the ocular tissue at the injection site of compression.

Preferably, one side of the clamping port is first brought into contact with the ocular surface, then is flipped with the contact part as a pivot point to allow the needle to penetrate the ocular tissue at the injection site, and continued to be flipped to allow the other side of the clamping port to contact the ocular surface, thus making ocular tissue form a bulge into the sleeve. The ocular surface may be an ocular surface, such as conjunctival tissue or other ocular tissue.

Preferably, the tissue thickness of the eye at the injection site is measured using one or more selected from optical coherence tomography (OCT), OCT-enhanced deep imaging (EDI-OCT), swept frequency OCT (SS-OCT), or ultrasound biomicroscopy (UBM).

Preferably, at least a portion of the substance in the medication container is delivered into the target tissue of the eye via the needle by pushing the pushrod.

Preferably, a volume of the medicament reflux in the sleeve is observed using an observation window, or an injection device with a transparent sleeve while pushing the pushrod to deliver at least a portion of the substance in the medicament container to the target tissue of the eye via the distal end of the needle observation window.

Preferably, as the ocular injection assembly is pressed to puncture the ocular tissue, the thrust assembly, in conjunction with the syringe, applies a force to the clamping port at the distal end of the sleeve, causing the ocular tissue surrounding the injection site to bulge into the sleeve.

Preferably, the thrust assembly exerts no more than 0.4N-6N, more preferably 1N-6N, more preferably 1-3N, to the clamping port at the distal end of the sleeve with the syringe cooperating with the ocular injection assembly when the ocular injection assembly is pressed to puncture the ocular tissue.

Preferably, the injection pressure within the medication container does not exceed 500 kPa when pushing the pushrod to deliver at least a portion of the substance of the medication container to the target tissue of the eye via the distal end of the needle.

Preferably, the intraocular pressure rises no more than 30 mmHg, more preferably no more than 20 mmHg, more preferably no more than 10 mmHg, when the pushrod is pushed to deliver at least a portion of the substance of the medication container to the target tissue of the eye via the distal end of the needle.

Alternatively, the present invention also provides an ocular injection device comprising a syringe 1, a needle 2, and a sleeve 3, with a clamping port 31 provided at the distal end of the sleeve 3, wherein the clamping port 31 is tightly fitted to the ocular tissues when injecting the eye to enable the ocular tissues to form a bulge towards the sleeve 1.

The syringe 1 further comprises a medication container 11, a pushrod 12 coupled to the medication container 11, and a needle 2 with its proximal end connected to the medication container 11, the distal end of the pushrod 12 being disposed within the medication container 11, the proximal end portion of the pushrod 12 being subjected to a force to move the distal end of the pushrod 12 within the medication container 11 for delivering at least a portion of the substance therein via the needle 2; a sleeve 3 being provided with a canal 32 and movably coupled to the proximal end of the needle 2, the distal end of the needle 2 being configured to pierce the eye tissue through the canal 32 of the sleeve 3 and the clamping port 31.

When the ocular injection device performs a clamping action, the sleeve 3 forms a bottom-sealed chamber 33 with the clamped ocular tissue.

The shape of the clamping port 31 may be circular, hexagonal, octagonal, square or irregular, and preferably circular. The minimum inner diameter of the clamping port 31 is 0.5 mm- 10 mm, preferably 1-6 mm, and more preferably 1 mm- 3 mm;

The length of the blade at the distal end of the needle 2 is less than 1100 micrometers, preferably less than 900 micrometers, further preferably less than 700 micrometers, more preferably ≤ 550 micrometers, and most preferably 250-550 micrometers.

The penetration force at the distal end of the needle 2 is <0.7N, more preferably <0.5N.

The force applied to the ocular tissue by the ocular injection device is capable of inducing an elastic deformation of the ocular tissue, preferably from 0.4N to 10N, more preferably from 2N to 6N, and most preferably from 3N to 5N.

The needle 2 of the ocular injection device comprises a needle hub 21 with its proximal end coupled to the distal end of the medication container 11 and the distal end coupled to the proximal end of the needle 2, and an adjusting assembly 4 is provided between the proximal end and the distal end of the needle hub 21, which cooperates with the sleeve 3 in adjusting a length of the needle 2 that extends outside the clamping port 31.

An adjustment release member 5 is provided between the needle hub 21 and the sleeve 3 adjustment release member configured for adjusting the length of the needle 2 extends outside the clamping port 31 and for pushing the needle 2 piercing into the ocular tissues via the distal clamping port 31 during puncturing action by the ocular injection device.

The injection device may comprise a thrust assembly 6 configured to apply a constant thrust force on the proximal end portion of the push rod 12.

The injection device may further comprise a second stop member 7 configured to selectively restrict movement of the push rod 12 with respect to the medication container 11, as well as to release the thrust assembly 6 when performing a drug injection.

The sleeve 3 is a transparent sleeve configured for observing the length of the needle 2 extending outside the clamping port 31, or\and configured to observe the amount of medicament reflux to the chamber 33.

Alternatively, the sleeve 3 may be provided with an observation window 34 configured for observing the length of the needle 2 outside the clamping port 31, or \and configured for observing the amount of medicament reflux in the chamber 33.

In summary, the following beneficial effects may be brought about by the above technical solutions:
1. By setting the clamping port as an annular end surface with an appropriate area of ocular tissues therein, a clear bulge can be formed inside the clamping port so that the medical solution can be delivered to the target site smoothly while effectively preventing reflux and spreading of the drug solution under the conjunctiva.
2. Provision of the adjusting assembly facilitates the adjustment of the length of the tip of the needle outside the clamping port and controlling the effective length of the tip of the needle, so that the effective dose of the drug is accurately delivered to the target tissue via the needle. The injection assemble of the present invention can be applied to different patients and can pierce different locations of the eye, such as being applied to the eyeball wall tissue for patients with abnormal scleral thickness.
3. The operation lever in the adjustment assembly is detachably connected, and the first stopper in the release assembly can be driven out of the release assembly, which facilitates one-handed operation by the operator and makes the operation more convenient.
4. The operation becomes more convenient by using the ocular injection assembly in conjunction with a syringe.
5. In the method of using the ocular injection device of the present invention, different injection assembly can be selected according to different patient conditions. The needle puncture is conducted after the peripheral tissues of the injection point form a bulge inside the sleeve, which facilitates the needle to reach the predetermined position and ensures the injection effect.
6. The leakage of drug from the piercing site can be effectively prevented, thereby improving the success rate of one-time puncture at a predetermined site. Even if reflux occurs due to operation or other reasons, the refluxed medication will go back to the sleeve through the clamping port, effectively avoiding the diffusion of the refluxed medication on the ocular surface as a result of injection failure.
7. The chamber inside the sleeve forms a sealed chamber. In case of reflux, the refluxed liquid is collected and stored in the sealed chamber. When separating the clamping port from the ocular tissue, the liquid will not easily leak out of the sealed chamber, preventing the liquid from dispersing onto the ocular tissue. Combined with the use of a transparent window and the setting of a scale, it is more conducive to accurately judging the reflux situation, as well as observing the volume of the refluxed liquid and estimating the amount of liquid injected.

### Brief description of drawings

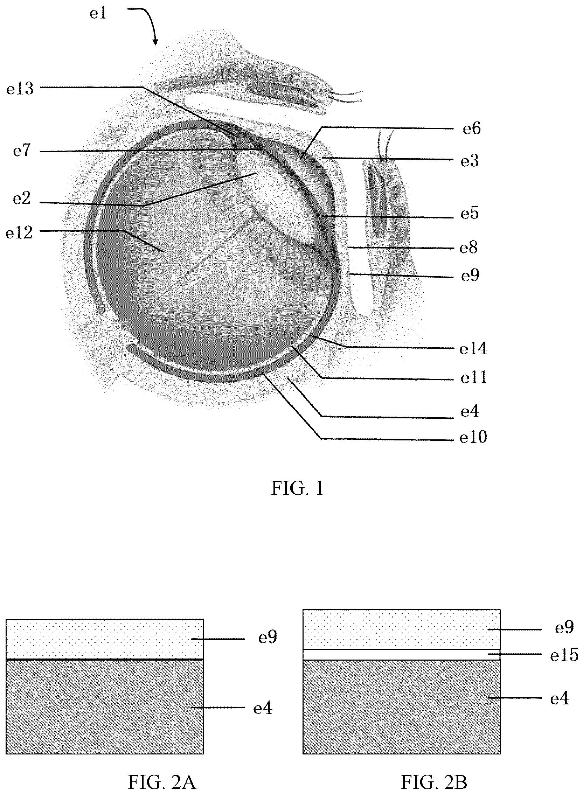

FIG. 1 shows a cross-section of a human eye.
FIG. 2A is a schematic diagram of the subconjunctiva without fluid, and FIG. 2B is a schematic diagram of the subconjunctiva with fluid.
FIG. 3A is a diagram showing the effect of no fluid in the suprachoroidal space, and FIG. 3B is a diagram showing the effect of no fluid in the suprachoroidal space.
FIGS. 4 to 18 are the accompanying drawings in Embodiments 1 to 9, in which:

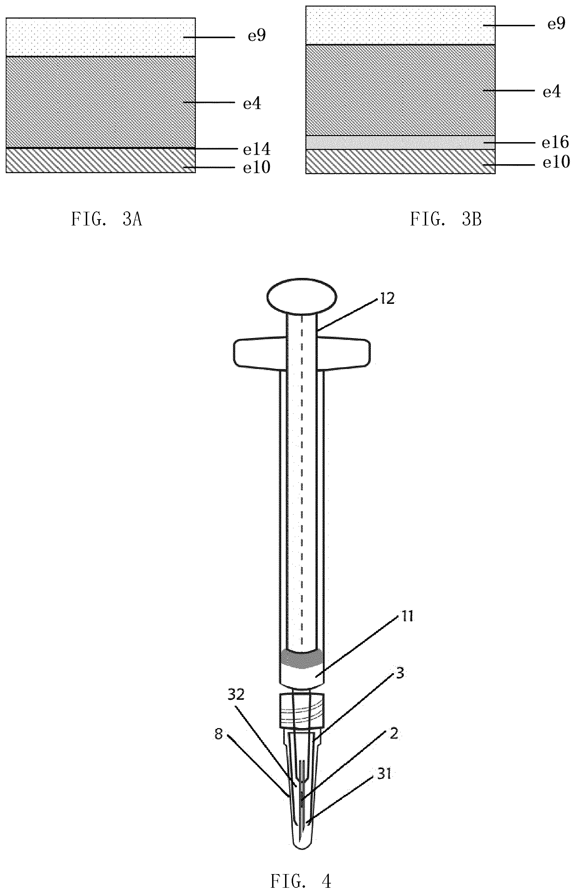

FIG. 4 is a schematic diagram of the structure of an ocular injection device.

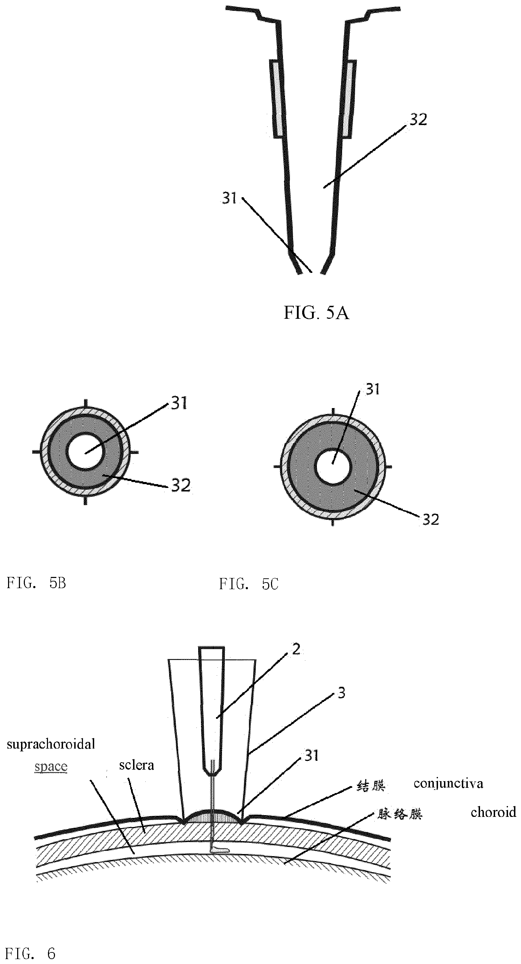

FIG. 5A is a front view of the sleeve shown in FIG. 4, FIG. 5B shows a top view thereof, and FIG. 5C shows an elevation view thereof.
FIG. 6 is a schematic front view showing the ocular tissue structure in the bulge formed by the ocular syringe of FIG. 4 and the ocular tissue.

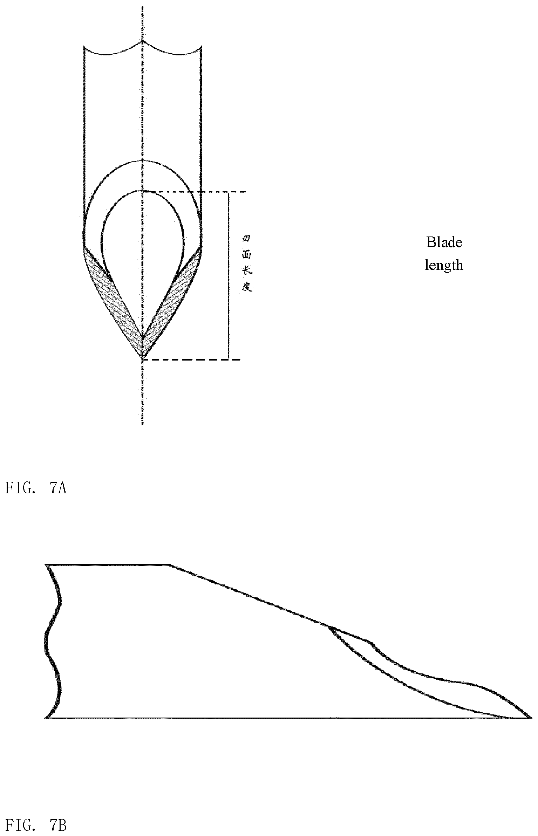

FIG. 7A is a front view of the blade at the distal end of the needle shown in FIG. 4, and FIG. 7B shows a side view thereof.

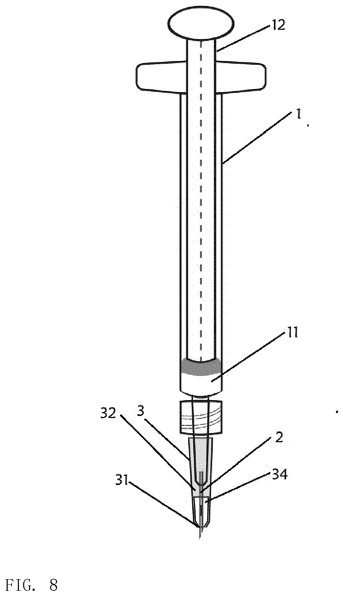

FIG. 8 is a schematic structural diagram of an ocular injection device provided with a bottom seal chamber and an observation window.

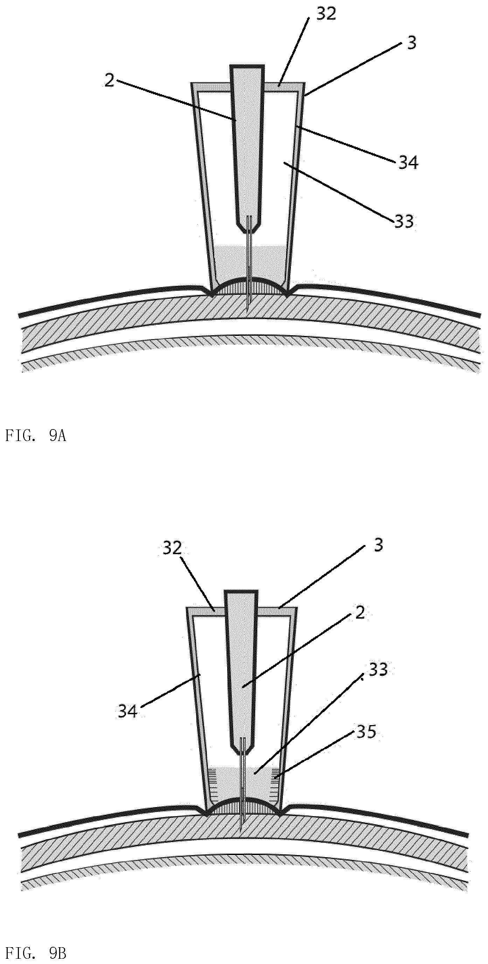

FIG. 9A is a schematic diagram of the sleeve shown in FIG. 8 with an observation window and a bottom sealing chamber, and FIG. 9B shows a schematic diagram of the sleeve shown in FIG. 8 with a volume scale.

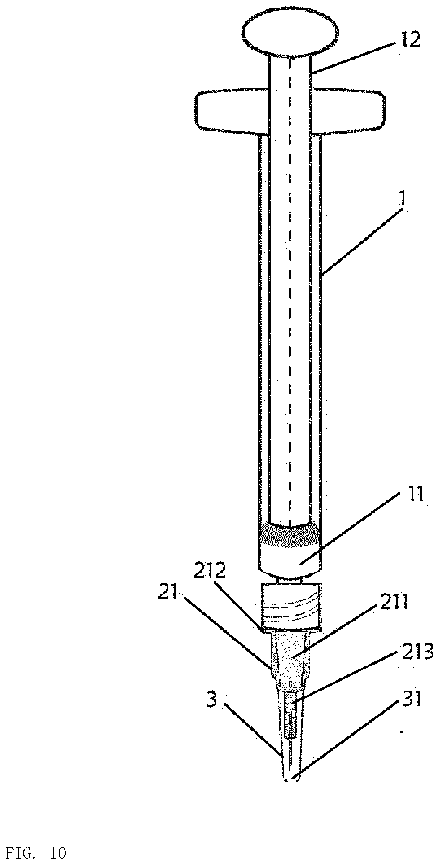

FIG. 10 is a schematic diagram of the structure of an ocular injection device comprising a needle hub.

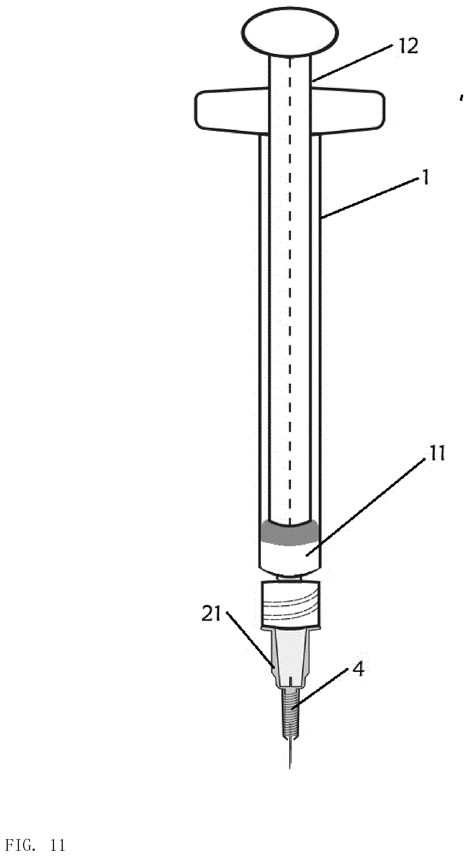

FIG. 11 is a schematic structural diagram of an ocular injection device provided with a threaded adjustment assembly.

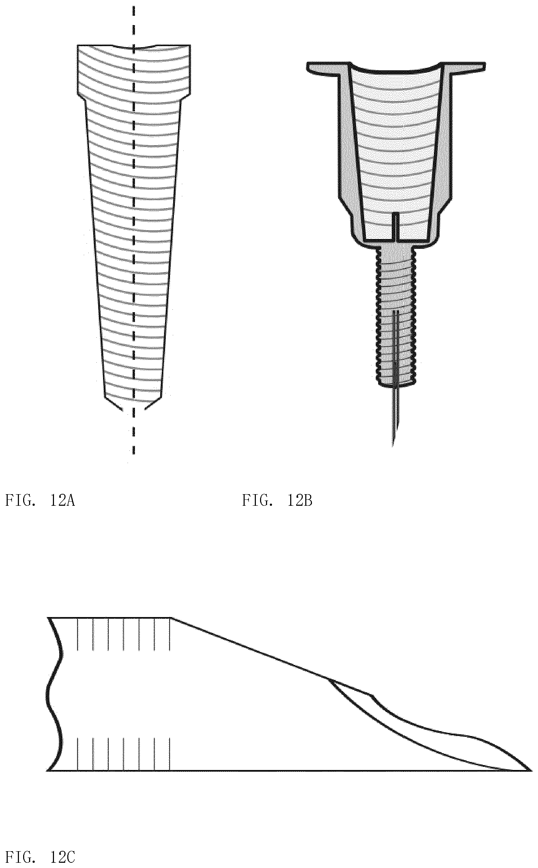

FIG. 12A is a schematic diagram of a sleeve structure with internal threads, FIG. 12B is a schematic diagram of a needle hub structure provided with external threads, and FIG. 12C is a schematic diagram of a distal portion of a needle with a volume scale.

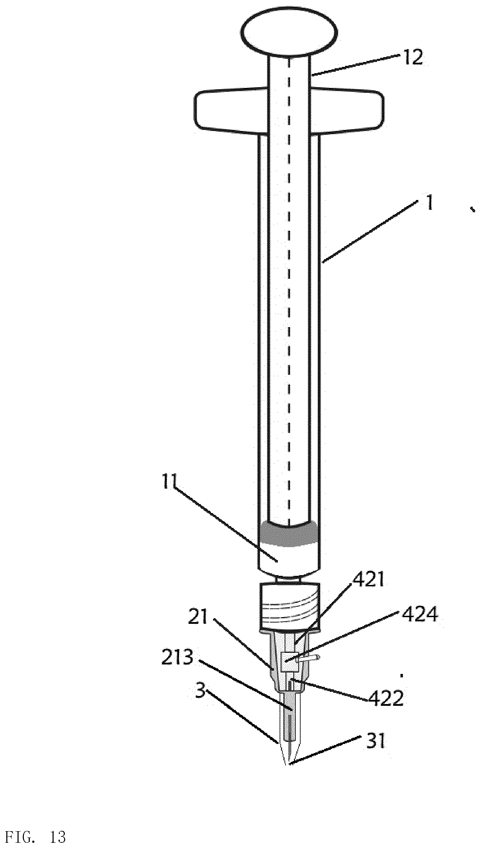

FIG. 13 is a schematic structural diagram of an ocular injection device provided with a telescoping rod adjustment assembly.

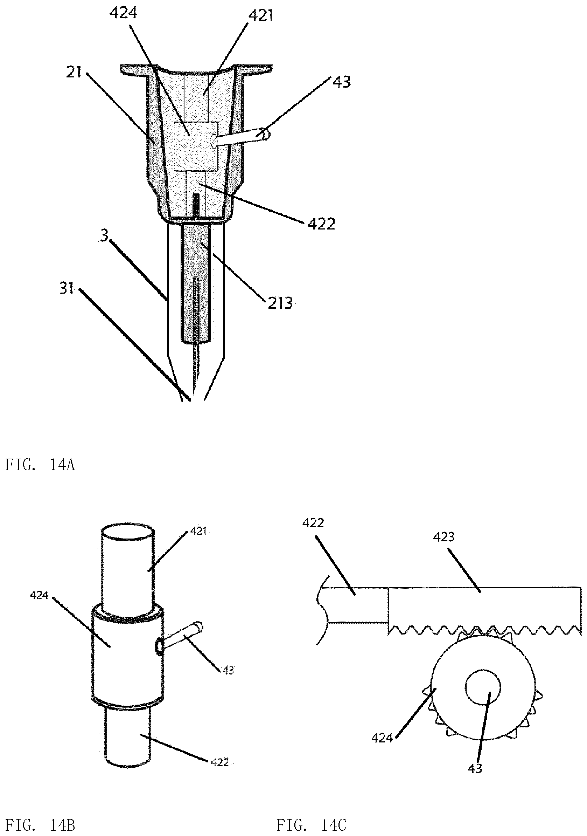

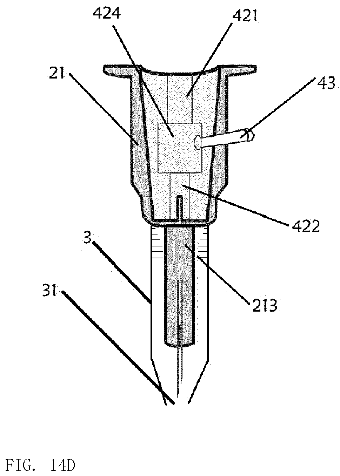

FIG. 14A is a schematic diagram of the structure of a needle provided with a telescoping rod adjustment assembly, FIG. 14B is a schematic diagram of the structure of the telescoping rod adjustment assembly, FIG. 14C is a schematic diagram of the internal structure of the telescoping rod adjusting mechanism, and FIG. 14D is a schematic diagram of the structure of a needle hub sleeve assembly with a volumetric scale.

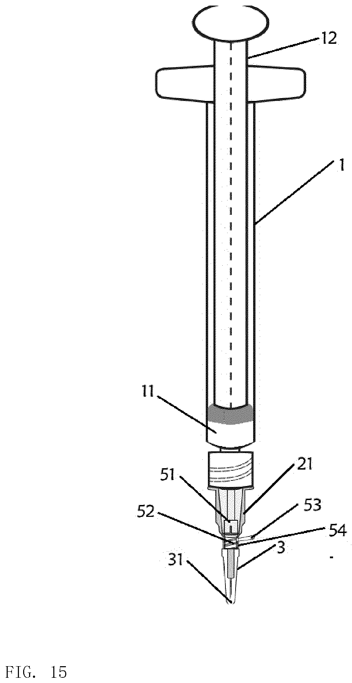

FIG. 15 is a schematic structural diagram of an ocular injection device having an adjustable release assembly.

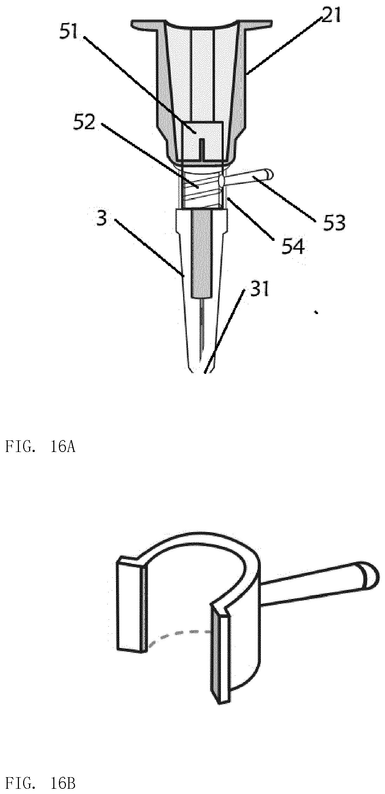

FIG. 16A is a schematic diagram of the structure of a needle having an adjustable release assembly, and FIG. 16B is a schematic diagram of the structure of a first stop member.

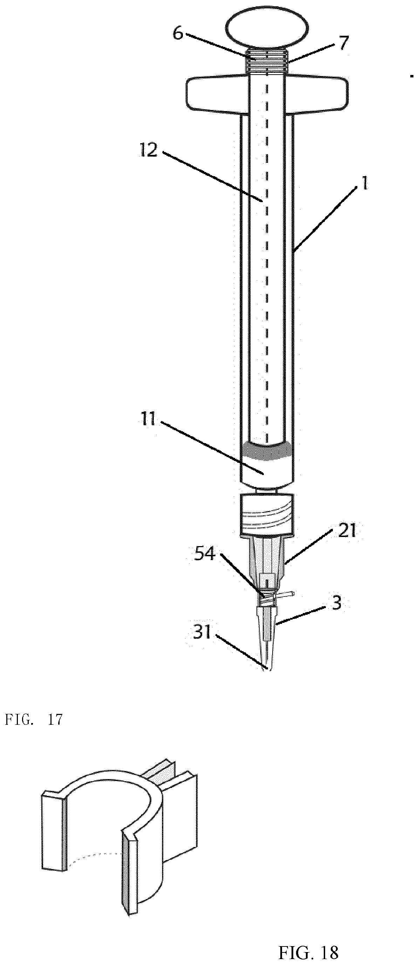

FIG. 17 is a schematic diagram of the structure of an ocular injection device comprising a thrust assembly.
FIG. 18 is a schematic diagram of the structure of the second stop member.
Reference signs in FIGS. 1 to 18 are explained as follows:

| | | |
|---|---|---|
| e1-eye | e2-lens | e3-cornea e4-sclera |
| e5-iris | e6-anterior chamber | e7-posterior chamber |
| e8-corneal rim | e9-conjunctiva | e10-choroid |
| e11-retina | e12-vitreous body | e13-ciliary body |
| e14-suprachoroidal | space | e15-fluid layer; |
| 1-syringe | 11-medication container | 12-pushrod 12 |
| 2-needle | 21-needle hub | 211-channel |
| 212-flange | 213-connecting portion | 214-ribs |
| 3-sleeve | 31-clamping port | 32-canal |
| 33-chamber | 34-observation window | 35-volume scale |
| 36-limit Hole | 4-adjustment assembly | 41-threads |
| 42-telescoping rod assembly | | 421-outer tube |
| 422-inner tube | 423-guideway | 424-drive mechanism |
| 43-first operating lever | | 5-adjustment release member |
| 51-telescopic assembly | | 52-release assembly |
| 53-second operating lever | | 54-first stop member |
| 6-thrust assembly | 7-second stop member | 8-needle protection cap |

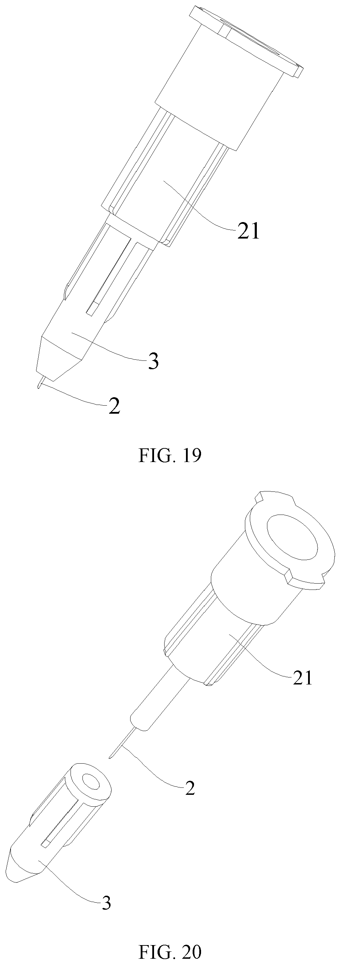

FIG. 19 is a schematic diagram of the structure of the ocular injection assembly of Embodiment 10.
FIG. 20 is a schematic diagram showing assembling of the ocular injection assembly of Embodiment 10.

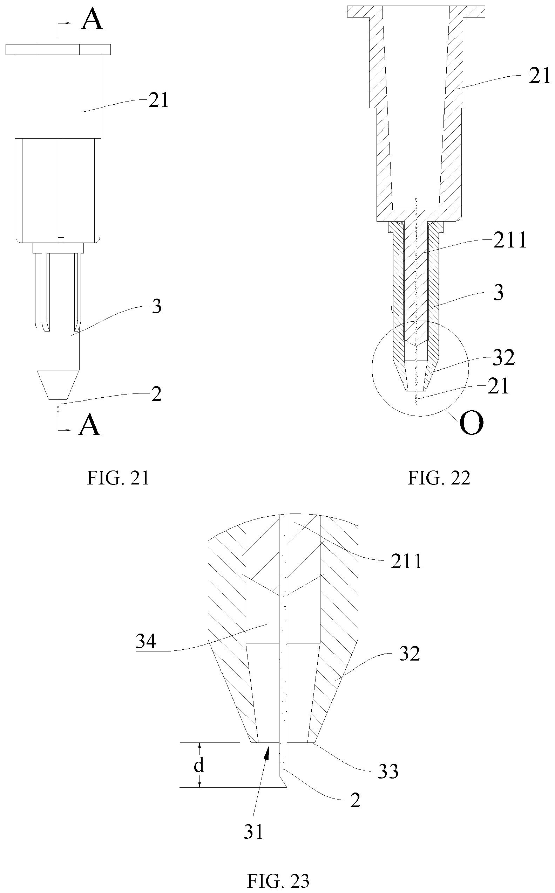

FIG. 21 is a schematic front view of the ocular injection assembly of Embodiment 10.
FIG. 22 is a sectional view at A-A of FIG. 21.
FIG. 23 is an enlarged schematic view at circle O of FIG. 22FIG., in which d is the length of the tip portion of the needle outside the portion of the clamping port.

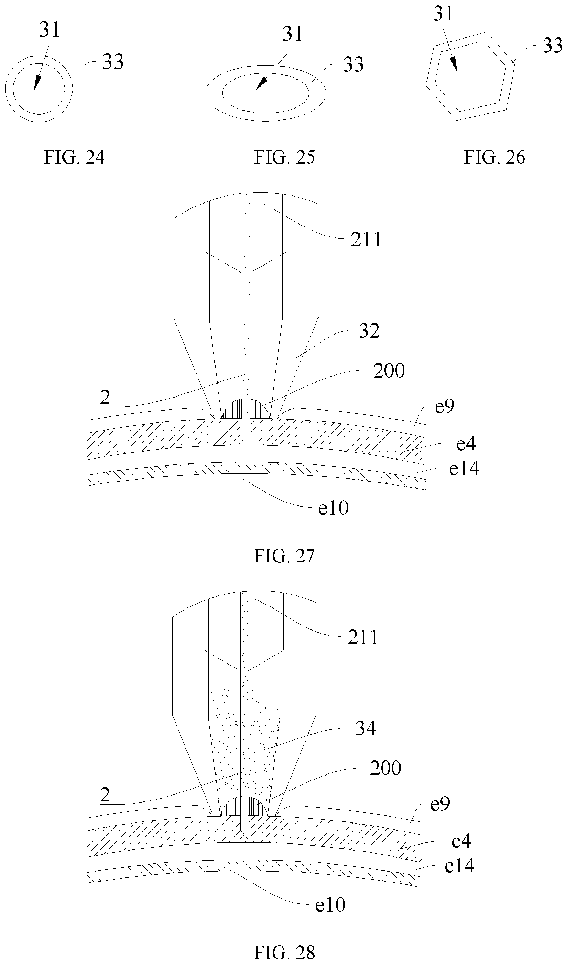

FIG. 24 is a schematic diagram of a circular clamping port with an annular end face.
FIG. 25 is a schematic view of an oval shaped clamping port with an annular end face.
FIG. 26 is a schematic diagram of a polygonal clamping port with an annular end face.
FIG. 27 is a schematic diagram of the clamping port in relation to the ocular surface when the ocular injection assembly is in use.
FIG. 28 is a schematic diagram of the clamping port in relation to the ocular surface when reflux occurs during use of the ocular injection assembly.

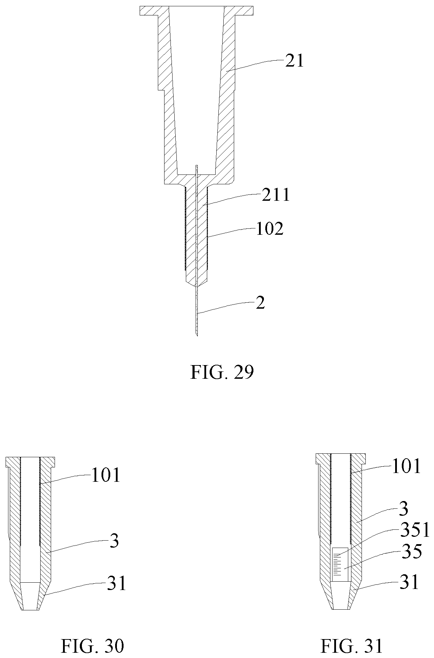

FIG. 29 is a schematic diagram of a sectional structure of the needle hub and needle of the injection assembly of Embodiment 11.
FIG. 30 is a schematic diagram of a sectional structure of the sleeve of the injection assembly of Embodiment 11.
FIG. 31 is a schematic diagram of a sectional structure of the sleeve of an alternative embodiment of the injection assembly of Embodiment 11.

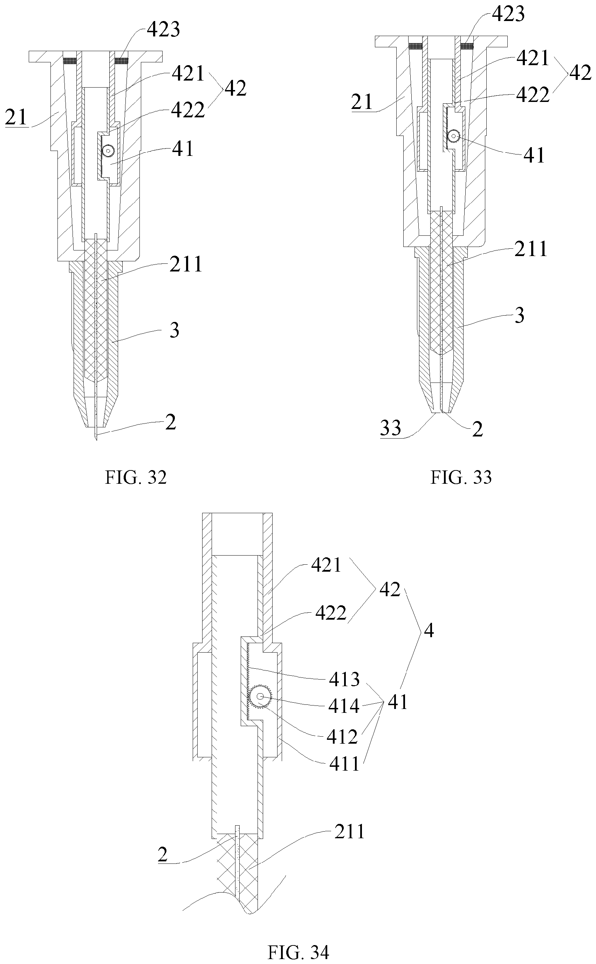

FIG. 32 is a schematic diagram of a sectional structure of the injection assembly of Embodiment 12.
FIG. 33 is a schematic diagram of a sectional structure of another state of the injection assembly of Embodiment 12.
FIG. 34 is a schematic diagram of the structure of the adjustment assembly of the injection assembly of Embodiment 12.

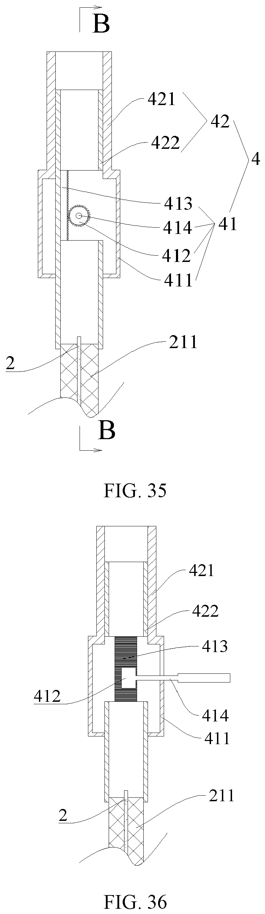

FIG. 35 is a schematic diagram of a sectional structure of an adjustment assembly of an alternative embodiment of the injection assembly of Embodiment 12.
FIG. 36 is a schematic view of the sectional structure at B-B of FIG. 35.

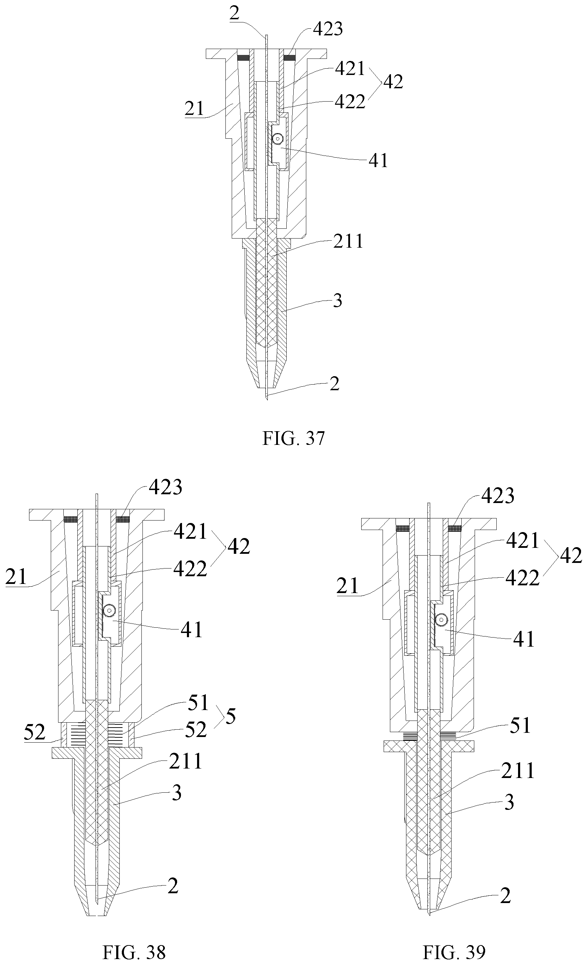

FIG. 37 is a schematic diagram of a sectional structure of an alternative embodiment of the injection assembly of Embodiment 12.
FIG. 38 is a schematic diagram of a sectional structure of the injection assembly of Embodiment 13.
FIG. 39 is a schematic diagram of a sectional structure of another state of the injection assembly of Embodiment 13.

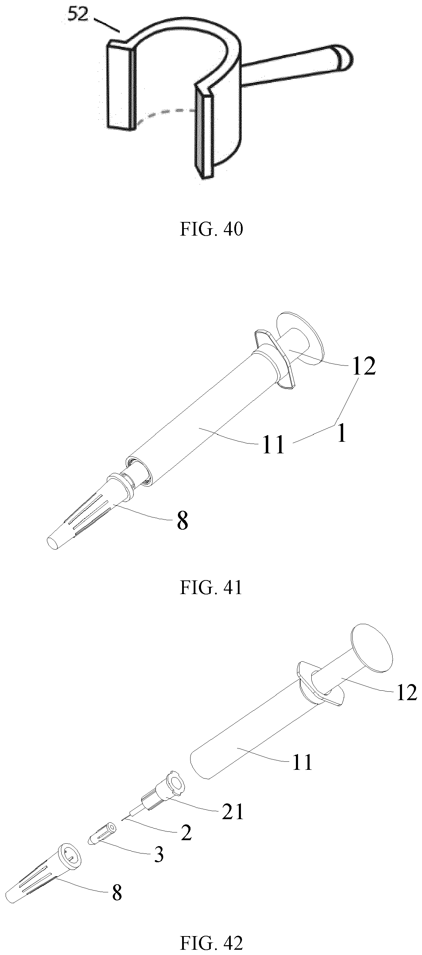

FIG. 40 is a schematic view of the structure of the first stop member of Embodiment 13.
FIG. 41 is a schematic diagram of the structure of the injection device of Embodiment 14.
FIG. 42 is an exploded view schematic of the injection device of Embodiment 14.

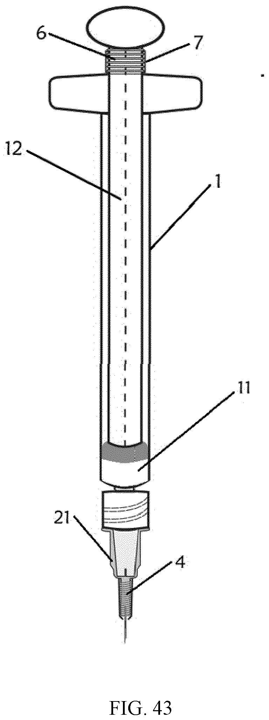

FIG. 43 is a schematic diagram of the structure of the injection device of Embodiment 15.

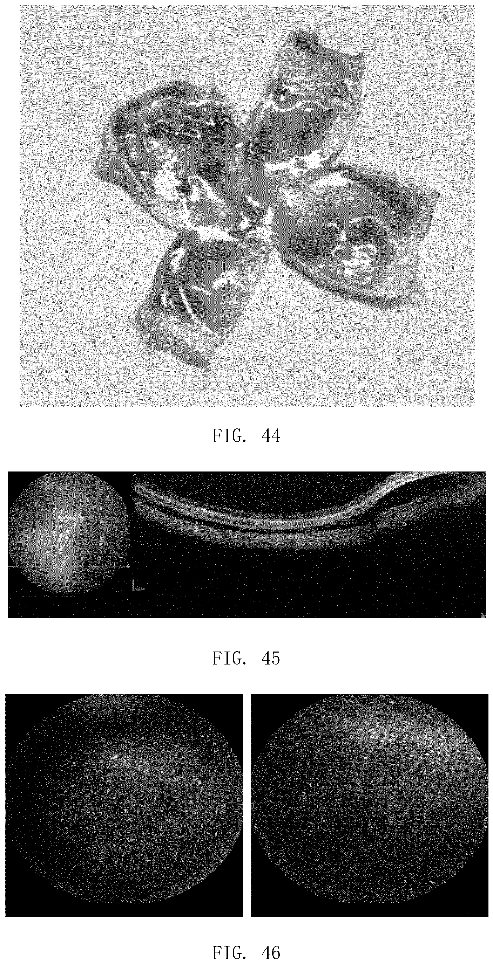

FIG. 44 is a picture of a section of fundus tissue from Test Example 1.
FIG. 45 shows the ICGA contrast results and OCT scan results in Test Example 3.
FIG. 46 shows the results of the fundus fluorescence assay in Test Example 3.

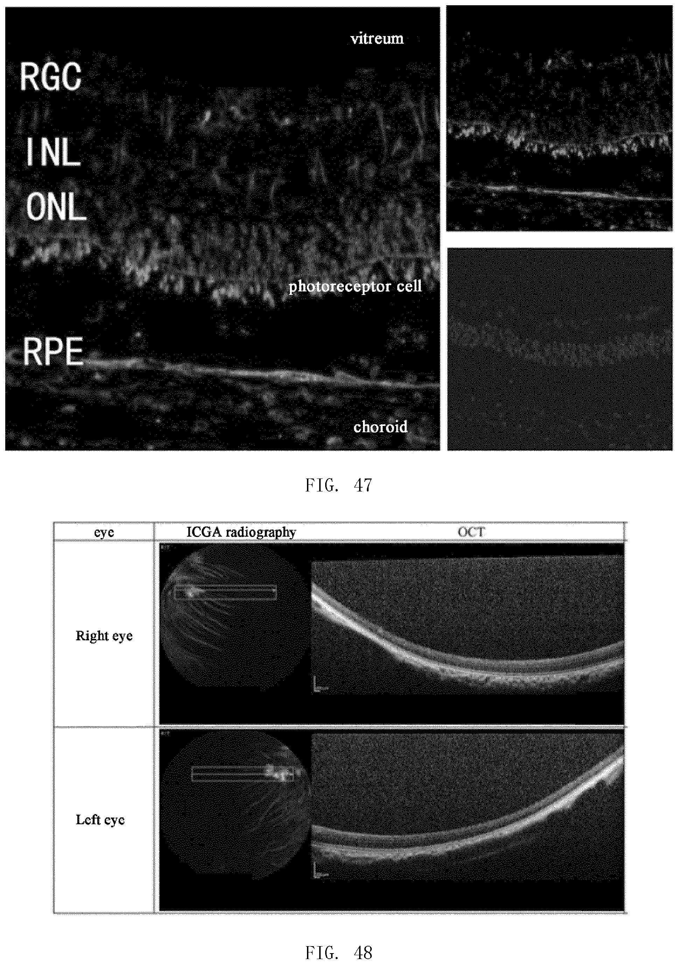

FIG. 47 shows the results of the expression of the injected reagent in retinal epithelial cells and photoreceptor cells in Test Example 3.
FIG. 48 shows ICGA contrast results and OCT scan results in Test Example 4.

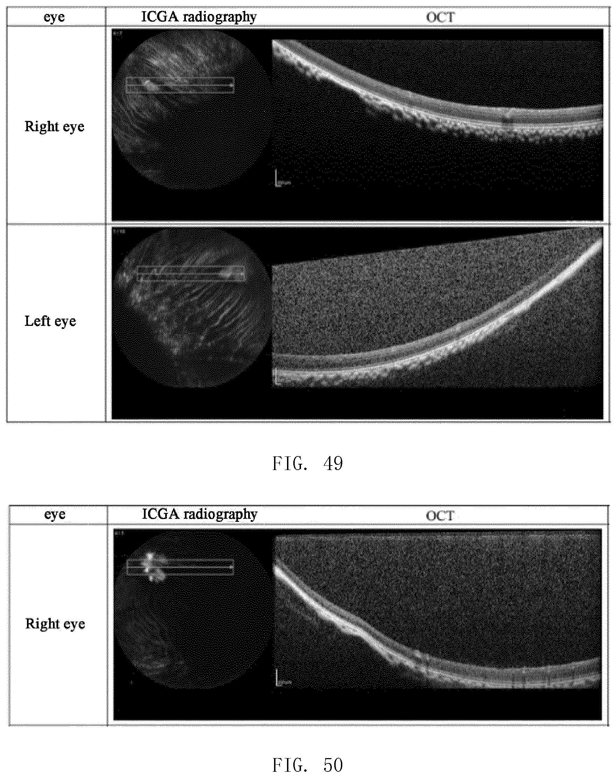

FIG. 49 shows ICGA contrast results and OCT scan results in Test Example 5.
FIG. 50 shows ICGA contrast results and OCT scan results in Test Example 6.

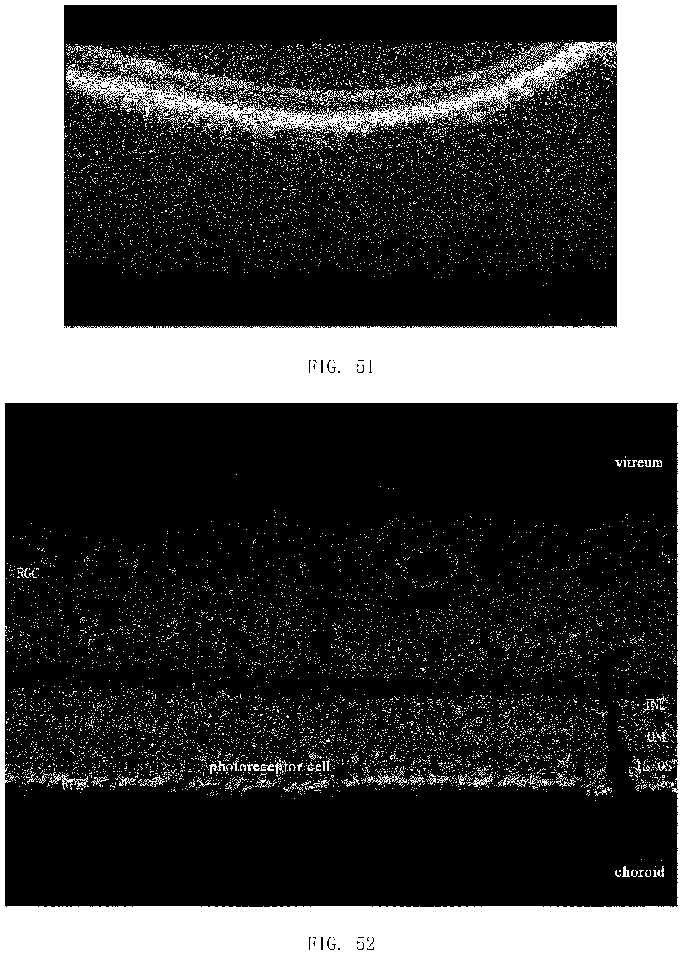

FIG. 51 shows the OCT scan results in Test Example 7.
FIG. 52 shows the results of the expression of the injected reagent in retinal epithelial cells, photoreceptor cells in Test example 7.

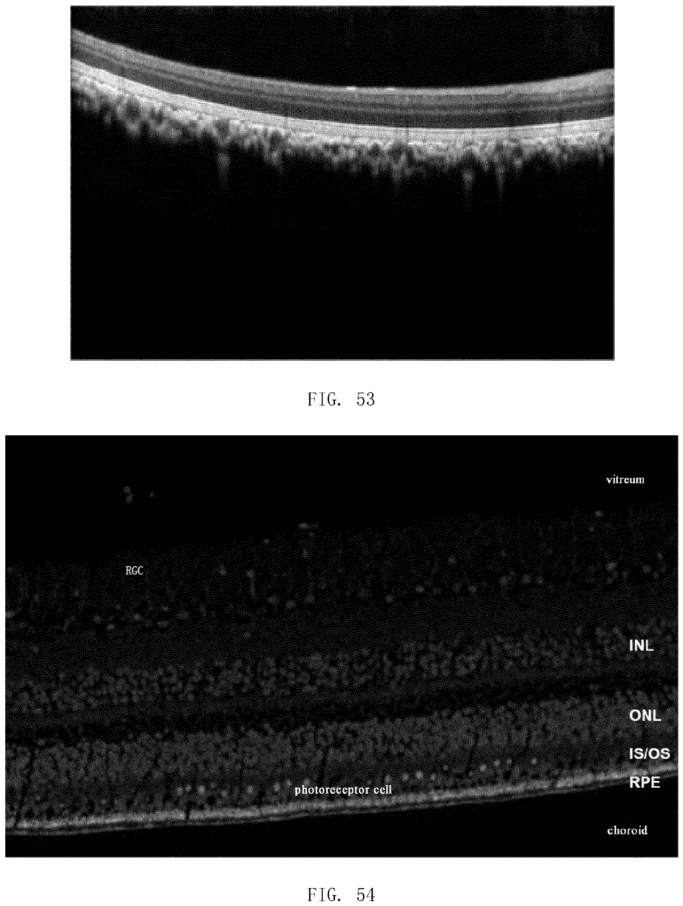

FIG. 53 shows the results of the OCT scan in Test example 8.
FIG. 54 shows the results of the expression of the injected reagent in retinal epithelial cells, photoreceptor cells in Test example 8.

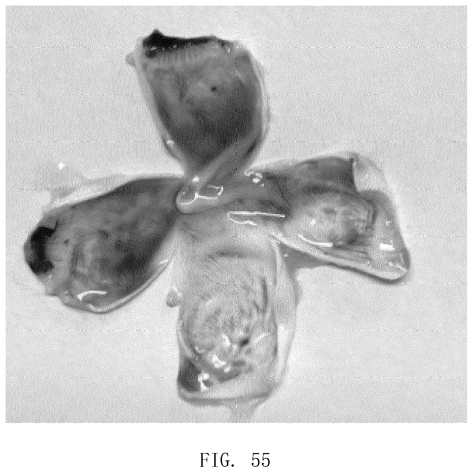

FIG. 55 is a picture of a section of fundus tissue from Comparative example 2.
Reference signs in FIGS. 19 to 55 are explained as follows:

| | | |
|---|---|---|
| 101-internally threaded section | | 102-externally threaded section |
| 200-bulge | 2-needle | 21-needle hub |
| 211-guide tube | 3-sleeve | 31-clamping port |
| 32-retracting section | 33-annular end surface | |
| 34-chamber | 35-observation window | 351-volume graduations |
| 4-adjustment assembly | 41-drive mechanism | 411-drive housing |
| 412-drive gear | 413-rack guide | 414-operating lever |
| 42-retractable rod assembly | | |
| 421-outer tube | 422-inner tube | |
| 5-release assembly | 51-resilient member | 52-first stop member |
| 6-thrust assembly | 7-second stop member | 8-needle protection cap |

### Detailed description

Embodiments of the present invention are described in detail below in conjunction with the accompanying drawings.

The injection site, needle gauge, blade length, blade penetration force, constant thrust and piercing application force of the thrust assembly as well as the needle gauge are described below:
1. Injection site
   Injection sites described herein include points in any area of supranasal, infranasal, supratemporal, infratemporal, etc., of the conjunctiva, between the iris rim and the corneal rim, approximately 3-9 mm from the corneal rim, approximately 4-8 mm from the corneal rim, approximately 4-7 mm from the corneal rim, approximately 6-8 mm from the corneal rim, approximately 7-8 mm from the corneal rim, approximately 4-5 mm from the corneal rim. A point that is approximately 3 mm from the corneal rim, or approximately 4mm, or approximately 5mm, or approximately 6mm, or about 7mm, or about 8mm may be elected.
2. Needle specifications
   The specification of needles can be selected from commercially available conventional injection needles such as 28G, 30G, 31G, 32G, 33G, 34G needle, or customized using conventional process of manufacturing the injection needle.

### Effective length of needle

Also named as the effective piercing length of the needle, which is the length of the needle extending out of the clamping port and is about 1400 microns or less, about 1300 microns or less, about 1200 microns or less, about 1100 microns or less, about 1000 microns or less, about 900 microns or less, about 800 microns or less, about 850 microns or less, about 700 microns or less, about 650 microns or less, about 500 microns or less, about 450 microns or less. In some embodiments, the needle effective length may be about 700 micrometers. In other embodiments, the effective length of needle may be about 750 microns, or about 800 microns, or about 850 microns, or about 900 microns, or about 950 microns, or about 1000 microns, or about 1100 microns, or about 1350 microns;

### 3. Blade length

A straight-line distance from the proximal inner edge of the needle wall at the distal outlet of the needle to the distal edge of the outer needle wall at the distal outlet of the needle, about 800 microns or less, about 700 microns or less, about 650 microns or less, about 600 microns or less, about 550 microns or less, about 500 microns or less, about 450 microns or less, about 400 microns or less, about 350 microns or less, about 300 microns or less, about 250 microns or less. In some embodiments, the length of the distal edge of the needle is about 550 microns. In other embodiments, the length of the distal edge surface of the needle is about 700 micrometers, or about 650 micrometers, about 600 micrometers, about 500 micrometers, about 450 micrometers, about 300 micrometers, about 250 micrometers.

### 4. Blade piercing force

The blade surface piercing force of the distal outlet port of the needle is about 0.7N or less, about 0.65N or less, about 0.5N or less, about 0.4N or less, about 0.3N or less, in order to facilitate defining arrival at a desired location (e.g., the suprachoroidal space and/or vitreous body) within the target tissue and to form a medicament delivery channel. In some embodiments, the blade piercing force may be about 0.5N, and in other embodiments, the blade penetration force or may be about 0.7N, or about 0.65 N, about 0.4N, about 0.3N.

### 5. Needle specifications

Needle Gauge: The distal end of the needle is typically constructed to be sharp, beveled cutting, or other forms capable of piercing the ocular surface (e.g., sclera). The needle employed may be of any suitable gauge, e.g., about 25 G, about 26 G, about 27 G, about 28 G, about 29 G, about 30 G, about 31 G, about 32 G, about 33 G, about 34 G, about 35 G, about 36 G. The needle wall may be of any suitable thickness. For example, in addition to the regular wall thickness (RW), the wall of the needle may be designed as thin wall (TW), super/ultra thin wall (XTW/UTW), or super thin wall (XXTW), which are well known to those skilled in the art. For example, the needle may be a fine gauge cannula or needle. In some embodiments, the needle may have a gauge between about 25G and about 36G. In other embodiments, the catheter may have a gauge between about 27G and about 35G. In additional embodiments, the needle may have a gauge between about 30G and about 33G.

Embodiments described herein relate to systems and devices for delivering a fluid (e.g., drug) into eye tissue. Additionally, the above embodiments relate to systems, devices, and methods that help the piercing of a delivery member (e.g., a needle) into the eye at a predetermined point of injection and/or help the effective metered injection of a drug into a target ocular tissue. The above embodiments also relate to systems, devices, and methods for avoiding the formation of a subconjunctival leakage channel around a delivery member (e.g., a needle) to avoid conjunctival and scleral gap diffusion during puncture to prevent diffusion of a substance and/or ocular fluid under the conjunctiva. The above embodiments also relate to systems, devices, and methods for forming a sealed chamber around a delivery member (e.g., a needle) during puncture to prevent diffusion of substances and/or ocular fluids around the ocular surface. The above embodiments also relate to systems, devices, and methods that are transparently structured or provided with an observation window around the sleeve to allow the operator to quickly and visually observe the needle length adjustment or the reflux of the substance and/or ocular fluid.

The "syringe" of the present invention is a conventional syringe assembly for ophthalmic use, comprising primarily a medication container 11, a pushrod 12 coupled to the medication container 11, and a needle 2 with its proximal end connected to the medication container 11. The distal end of pushrod 12 is placed within the medication container 11 and its proximal end is subjected to a force to move the distal end of the pushrod 12 within the medication container 11 to deliver at least a portion of the substance in the medication container 11 via the needle 2.

### Eye Structure

FIGS. 1-3 show a human eye for reference (of which FIGS. 2 and 3 are sectional views). Although the drawings of this specification relate to specific regions of the eye, it is understood by those skilled in the art that the drawings of specific regions of the eye in the drawings of the present application do not constitute the whole eye, but rather are intended only as specific embodiments applicable to the present invention so that those skilled in the art may understand the embodiments thereof. Wherein eye e1 comprises an anterior section (the portion of the eye anterior to the lens e2 (and including the lens e2)) and a posterior section (the portion of the eye posterior to the lens e2). The anterior section is bounded by cornea e3 and lens e2, and the posterior section is bounded by sclera e4 and lens e2. The anterior section includes an anterior chamber e6 between iris e5 and cornea e3 and a posterior chamber e7 between lens e2 and iris e5. Cornea e3 and sclera e4 together form the corneal limbus at their junctional positions e8. the exposed portion of sclera e4 in the anterior section is the conjunctiva e9 to protect the eye. Below sclera e4 are choroid e10 and retina e11, collectively known as the retinochoroidal tissue. Vitreous body e12 is between the ciliary body e13 and the retina e11. The loose connective tissue or potential space between choroid e10 and sclera e4 is called suprachoroidal space e14. As shown in FIG. 2, conjunctiva e9 is specifically a soft, smooth, and elastic mucous membrane covering the inside of the upper and lower eyelids and in front of the eyeball, and is a transparent membrane formed by a complex columnar epithelium and a small amount of connective tissue, and a small number of mucous glands, which can secrete mucus and make the surface of the eyeball smoother. Sclera e4 is divided into three layers (surface layer, stroma, and brown-black plate layer). The superficial layer is loose connective tissue, and the stromal and brownish-black plate layers are composed of dense connective tissue and elastic fibers, rendering sclera e4 dense and tough. During injection, if the drug leaks, it tends to return to the subconjunctiva and diffuse to form the fluid layer e15, as shown in FIGS.FIGS. 2A and 2B.

As shown in FIG. 3A, there is not a distinct gap existing in suprachoroidal space e14 in the absence of fluid and/or tissue separation, which is a potential space between sclera e4 and choroid e10. As shown in FIG. 3B, in the presence of fluid e16 in the suprachoroidal space e14, a distinct gap appears with sclera e4 above and choroid e10 below. Thus, suprachoroidal space e14 can be made visible in the region when fluid or other material accumulates between choroid e10 and sclera e4. Thus fluid accumulation is intentionally created by delivering, injecting and/or transfusing drug concoctions into the suprachoroidal space to further cause and/or expand the separation of the choroid from the sclera to form the suprachoroidal space.

The injection site location of either the ocular injection devices and/or methods of the present invention is in a region of 6-8 mm from the corneal rim, e.g., supranasal, infranasal, supratemporal, infratemporal, etc. An operator can confirm the injection site (7-8 mm from the corneal limbus) by measuring the distance using ophthalmic calipers. In this manner, the drug can be introduced (e.g., via a needle) into the suprachoroidal space from the injection site and can be pushed into the suprachoroidal space away from the insertion site.

As shown in FIG. 7, the length of the distal edge of the needle in the present invention is the maximum distance between the opening in the inner wall of the needle and the tip of the tip.

The term "distal" or "anterior" of the invention refers to the end near the eye tissue, and "proximal" or "posterior" refers to the end near the operator (e.g., physician or nurse). "proximal" or "posterior" refers to the end near the operator (e.g., a physician or nurse).

### Embodiments

### Embodiment 1:

An ocular injection device of this embodiment is shown in FIGS. 4-6. The ocular injection device comprises a syringe 1, a needle 2, and a sleeve 3. the distal end of the sleeve 3 is provided with a clamping port 31. When injecting into the eye, the clamping port 31 is tightly close-fitted to the ocular tissue to enable the ocular tissue to form a bulge towards the inside the sleeve 3.

The syringe 1 comprises a medication container 11 and a push rod 12. The medication container 11 is coupled to the proximal end of needle 2 coupled to push rod 12, and the distal end of the push rod 12 is disposed within the medication container 11. A force is applied on the proximal end of push rod 12 to move the distal end of push rod 12 within the medication container 11 to deliver at least a portion of the substance in the medication container 11 via the needle 2.

The sleeve 3 is provided with a canal 32 and is movably connected to the proximal end of needle 2. The distal end of needle 2 is configured to pierce the eye tissue via clamping port 31 through canal 32.

The ocular injection method of the present embodiment comprises, inter alia:
A first step of measuring the distance with ophthalmic calipers to confirm the injection site;
A second step of applying a force to a side of clamping port 31 of the sleeve 3 via the syringe 1 to form a pivot point on the conjunctival surface with the side of clamping port 31;
A third step of pivoting the clamping port 31 around the pivot point toward the other side of clamping port 31 so that the distal end of needle 2 pierces the conjunctival tissue at the injection site;
A fourth step of continuously pivoting the clamping port 31 around the pivot point to make the other side of clamping port 31 attach the conjunctival surface, so that clamping port 31 grips the conjunctival tissue of the eye to form a bulge into sleeve 3, and the distal end of needle 2 is pressed into the target tissue of the eye at the injection site; and
A fifth step of administration to inject the drug to reach the site of administration.

This embodiment also provides another method of ocular injection comprising:
A first step of measuring the distance with ophthalmic calipers to confirm the injection site;
A second step of placing clamping port 31 in perpendicular to the ocular surface of the injection site;
A third step of piercing the distal end of needle 2 into the conjunctival tissue at the injection site and making clamping port 31 contact the ocular surface at the injection site;
A fourth step of applying a force is applied to the ocular tissue at the injection site via the syringe 1 of the ocular injection device in conjunction with sleeve 3, so that the conjunctival tissue held by the clamping port 31 forms a bulge towards inside of sleeve 2, and the distal end of the needle 2 is pressed into the target ocular tissue at the injection site; and
A fifth step of administration to inject the drug to reach the site of administration.

The needle 2 of this embodiment can either be selected from commercially available conventional ophthalmic needles such as 31G and 32G needles, or customized and processed through the conventional process of making injecting needles.

The needle and the sleeve can be produced in a matching fashion. Needle sleeve assemblies with different sizes can be customized according to the thickness between the ocular surface of the point of injection to the suprachoroidal space in which medication is to be delivered. The different sizes depend on the length of the portion of needle 2 outside the clamping port 31, which may range from 500 to 2,000 microns, such as 700 microns, 800 microns, 900 microns, 1,000 microns, 1,100 microns, 1,200 microns, 1,300 microns, and so forth, for selection by the operator.

In order to avoid abrasion or laceration of the operator by the distal end of needle 2, the ocular injection device in this embodiment is further provided with a needle protection cap 8 configured to be movably connected to the sleeve 3 or the needle hub 21. Operator removes the needle protective cap 8 during use and install it on the needle after use for recycling it together with other components of the injection device.

A 700-micron needle sleeve assembly was selected to perform rabbit ocular suprachoroidal space injections according to the ocular injection method of the present embodiment, and it was found that the ocular injection device provided in the present embodiment not only was successful in a one-time puncture to deliver the drug to the target tissues of the eye, but also significantly reduced the subconjunctival leakage of the drug solution.

Through the study of the force applied to the ocular tissues by the ocular injection device, it is found that the force applied to the ocular tissues by the ocular injection device according to the embodiments should not be too large or too small. Too small a force cannot cause elastic deformation of the conjunctival tissue to form a bulge, and too large a force may damage the conjunctival tissue. A force applied between 0.4N and 10N can not only cause the conjunctival tissue to form a bulge in the sleeve 3, but also ensure that the bulge of the conjunctival tissue is automatically restored after completion of the injection, and significantly reduce the leakage of the liquid under the conjunctiva. When the applied force is controlled at a range from 2N to 6N, there is no leakage of drug under the conjunctiva.

Through the study of the shape of the clamping port 31, it is found that various shapes of the clamping port 31 of the ocular injection device, such as round, hexagonal, octagonal, square or irregular shapes, can realize the purpose of the present invention. Among them, when the clamping port 31 of the ocular injection device is round, it is not only more conducive to avoiding damage to the conjunctiva, but also more conducive to the recovery of ocular tissues during the injection process. When the inner diameter of the clamping port 31 is controlled at 0.5mm - 10mm, the conjunctival tissue forms an arch-shaped raised structure, so that the needle of the ocular injection device is pierced through the raised top of the ophthalmic tissue to reach the ophthalmic drug delivery site, thus making the needle more fixed on the conjunctival tissue at the injection site, and making the injection at the identified injection site simpler. When the inner diameter of the clamping port 31 is limited at 1-6 mm, the arch-shaped bulge structure is better, and the injection effect is best when the inner diameter of the clamping port 31 is 1 mm - 3 mm.

### Embodiment 2

The ocular injection device of this embodiment is detailed in FIGS. 7A-7B, and the length of the blade at the distal end of needle 2 is 700 micrometers.

The needle of this embodiment can be processed by the following process:
Step 1: Use seamless welding machine to wind the stainless-steel bar into a tube by laser welding, and then pull it thinly into 31G caliber stainless steel capillary tube through wall reduction machine, pipe drawing machine, straightening machine and other equipment;
Step 2: Cut the capillary to fixed length using a tube cutter;
Step 3: Fix the capillary by arranging it with a needle setting machine;
Step 4: A first sharpening at a set angle of 18° is carried out using a sharpening machine, followed by a second and a third sharpening at an angle of 35°. Stop sharpening when the length of the sharpened edge reaches 700 microns;
Step 5: Cleaning;
Step 6: Conduct needle assembly in a 100,000-class workshop.

The penetration force of the needle blade prepared by the above process was less than 0.4N according to the test by needle penetration experiments.

### Embodiment 3

The ocular injection device of this embodiment is detailed in FIGS. 7A-7B, and the length of the blade at the distal end of the needle 2 is 550 micrometers.

The needle of this embodiment can be processed by the following process:
Step 1: Use seamless welding machine to wind the stainless-steel bar into a tube by laser welding, and then pull it thinly into 31G caliber stainless steel capillary tube through wall reduction machine, pipe drawing machine, straightening machine and other equipment;
Step 2: Cut the capillary to fixed length using a tube cutter;
Step 3: Fix the capillary by arranging it with a needle setting machine;
Step 4: A first sharpening at a set angle of 22° is carried out using a sharpening machine, followed by a second and a third sharpening at an angle of 35°. Stop sharpening when the length of the sharpened edge reaches 550 microns;
Step 5: Cleaning;
Step 6: Conduct needle assembly in a 100,000-class workshop.

The penetration force of the needle blade prepared by the above process was less than 0.5N according to the test by needle penetration experiments.

### Embodiment 4

The ocular injection device of this embodiment is detailed in FIGS. 7A-7B, and the length of the blade at the distal end of the needle 2 is 250 micrometers.

The needle of this embodiment can be processed by the following process:
Step 1: Use seamless welding machine to wind the stainless-steel bar into a tube by laser welding, and then pull it thinly into 32G caliber stainless steel capillary tube through wall reduction machine, pipe drawing machine, straightening machine and other equipment;
Step 2: Cut the capillary to fixed length using a tube cutter;
Step 3: Fix the capillary by arranging it with a needle setting machine;
Step 4: A first sharpening at a set angle of 30° is carried out using a sharpening machine, followed by a second and a third sharpening at an angle of 35°. Stop sharpening when the length of the sharpened edge reaches 250 microns;
Step 5: Cleaning;
Step 6: Conduct needle assembly in a 100,000-class workshop.

The penetration force of the needle blade prepared by the above process was less than 0.7N according to the test by needle penetration experiments.

When the ocular injection devices provided in embodiments 2-4 of the present invention are used for suprachoroidal space injection in accordance with the injection method of Embodiment 1, it was found that not only reflux of the medicament did not occur, but also less puncture force is needed to be exerted by the operator, and it is more convenient to operate, while reducing the safety risk of the patient's ocular tissues due to puncture.

The edge surface of the distal end of needle 2 of the present invention can be machined into the distal end of needle 2 of Embodiments 2-4 by employing a single or multiple cuts, in addition to being machined into a three-sided structure by the three-knife cutting process provided in the above embodiments.

### Embodiment 5

The ocular injection device of this embodiment is shown in FIGS. 8-9, wherein the ocular injection device performs a clamping action, wherein sleeve 3 forms a bottom-sealed chamber 33 with the clamped ocular tissue.

Sleeve 3 is a transparent structure, or an observation window 34 is provided on the wall of sleeve 3, and the operator observes the reverse osmosis of the medicament through the transparent sleeve 3 or the observation window 34.

Injecting in accordance with the suprachoroidal space injection method of Embodiment 1, the refluxed medicament produced by the ocular injection device of the present embodiment is automatically refluxed into the bottom sealed chamber 33 formed by the sleeve 3 and the clamped ocular tissues via the injection port formed at the injection site. The operator can observe the refluxing medicament through the sleeve 3 or the observation window 34 on the sleeve 3, quickly assess whether the injection is successful or not, and quickly take remedial measures accordingly. For example, the operator, without removing the needle 2, increases the penetration force to make the distal end of the needle 2 penetrate the sclera; the operator, without removing needle 2, can adjust the length of needle 2 outside the clamping port 31 at the distal end of sleeve 3 through the adjustment assembly 4 of the present invention, and drive the distal end of the needle 2 to penetrate the sclera.

Meanwhile, this embodiment of the ocular injection device collects the reflux liquid centrally in the chamber 33, avoiding the reflux liquid from spreading around on the ocular surface, and greatly reducing the difficulty of cleaning up the ocular surface during the injection process or after the injection is completed.

In order to facilitate the operator in determining the amount of medicament reflux so as to assess whether this injection is successful, the embodiment provides a medicament reflux volume scale 35 in the observation window 34 or the transparent sleeve 3, and provides a warning limit in the scale, so as to remind the operator that the injection has failed and that the injection should be stopped.

### Embodiment 6

An ocular injection device is shown in FIG. 10, which comprises a needle hub 21 with a proximal end coupled to a distal end of a medication container 11 and a distal end coupled to a proximal end of a needle 2. The needle hub 21 is provided with a channel 211 to deliver at least a portion of substance from the medication container 11 to needle 2 via channel 211. The proximal and distal ends of the needle hub 21 are provided with a flange 212 configured to be coupled to sleeve 3.

The distal end of needle hub 21 is provided with a connecting portion 213 fixedly connected to the proximal end of needle 2. This embodiment allows the proximal end of needle 2 to be inserted through connecting portion 213 and to be fixed by sealing between the connecting portion 213 and the proximal end of the 2 by means of dispensing glue.

Flange 212 is provided with ribs 214 to allow an operator to easily grip needle hub 21 when installing the same.

The ocular injection device of this embodiment not only secures the needle in the connecting portion 213 to avoid the distal end of needle 2 from wobbling during puncture caused by bending of needle 2, but also facilitates the installation by the operator and makes it easier to use adjusting assembly 4 of the present invention to adjust the length of the distal end of needle 2 outside the clamping port 31 of the sleeve 3.

### Embodiment 7

The ocular injection device of this embodiment is shown in FIGS. 11-14, with an adjustment assembly 4 provided between the proximal and distal ends of needle hub 31, which cooperates with sleeve 3 to adjust the length of needle 2 outside the sleeve 3 clamping port 31.

The adjustment assembly 4 is provided with threads 41 in the outer wall of the distal portion of needle hub 21 and the inner wall of sleeve 3 respectively, and the length of the distal end of needle 2 outside the clamping port 31 is adjusted by rotating sleeve 3 through threads 41.

The adjustment assembly 4 is provided with a telescopic rod assembly 42 and a first operating lever 43. The telescopic rod assembly 42 includes an outer tube 421, an inner tube 422, a guide rail 423, and a drive mechanism 424. The outer tube 42 is connected to the needle hub 21, and the inner tube 422 is connected to a connecting portion 313. The guide rail 423 is mounted in the outer tube 421, and the inner tube 422 moves axially on the guide rail 42 driven by the drive mechanism 434.

One end of the first operating lever 43 is movably connected to the driving mechanism 434, and the other end is fixed on the outer side of the sleeve 3. By acting on the outer side of the sleeve 3, the first operating lever 43 drives the driving mechanism 434 to drive the inner tube 422 to move axially on the guide rail 423, so as to realize the free adjustment of the length of the needle 2 outside the clamping mouth 31.

The drive mechanism 434 of the telescoping rod assembly 42 is a linear drive mechanism, which can be driven by the first operating lever 43 through a conventional screw, a rack and pinion, a ball screw, or a pneumatic cylinder to adjust the length of needle 2 outside clamping port 31.

The operator can measure the length of needle 2 outside clamping port 31 adjusted by the adjusting assembly 4 by using ophthalmic calipers. This embodiment of the adjustment assembly 4 has the advantage of a flexible and adjustable length of the exposed distal end of needle 2, and may be suitable for patients with abnormalities in the thickness of ocular wall tissue, such as sclera.

Meanwhile, in order to facilitate the operator to more accurately observe and adjust the length of the needle 2, this embodiment is also provided with a volume scale line of different variables on the outer wall of transparent sleeve 3 or observation window 34. When manufacturing adjustment assembly 4 of this embodiment, a starting line for length adjustment of needle 2 can be provided on the outer thread of connecting portion 313, aligned with the starting line of the scale set on the outer wall of the transparent sleeve or the observation window, and the distal end of needle 2 can be aligned with the clamping port of the sleeve. The operator actuates the adjustment assembly to make an axial movement of the distal end of the needle towards the sleeve gripping port, so as to realize a precise adjustment of the length of the portion of needle 2 outside clamping port 31 without using ophthalmic calipers for measurement.

This embodiment also provides volume scale lines of different variables at the distal end of needle 2. The volume scale lines may be labeled by laser marking. When the volume scale line at the distal end of needle 2 is aligned with the clamping port, the length of the distal end of needle 2 outside clamping port 31 can be measured without using ophthalmic calipers.

### Embodiment 8

The ocular injection device of this embodiment is shown in FIGS. 15-16. An adjustment release member 5 is provided between the needle hub 21 and the sleeve 3. The adjustment release member 5 is configured to adjust the length of the needle 2 outside the distal clamping port 31 of the sleeve 3, and to push the distal end of the needle 2 to pierce into the target tissue of eye via the distal clamping port 31.

The adjustment release member 5 includes a telescoping assembly 51, a release assembly 52, and a second operating lever 53. the telescoping assembly 51 in this embodiment is the same as the telescoping rod assembly of Embodiment 7, and the second operating lever 53 is the same as the first operating lever of Embodiment 7.

Release assembly 52 is connected to flange 312 of needle hub 21 at one end and to the proximal end of sleeve 2 at the other end. The other end of second operating lever 53 connected to the sleeve is provided with a first stop 54, which is stuck between needle hub 21 and sleeve 3 via a limit hole 36 provided in sleeve 3. Release assembly 52 may be released by removing second operating lever 53 and its first stop to push the distal end of needle 2 into the ocular tissue via clamping port 31.

Release assembly 52 may be any of a reset spring, a compressed gas container, or a container containing propellant.

In manufacturing the adjustment release member 5 of this embodiment, the first stop 54 can be made as tab, slot, ring, or pawl, which can restrict the release assembly 52 from releasing the pull of the sleeve toward the proximal end of the needle hub to drive the distal end of the needle 2 toward the clamping port 31 for piercing into the ocular tissues. To release the tension of the release assembly 52, the operator removes the first stop 54 by pulling out the second operating lever 53.

The operator can adjust the length of the portion of needle 2 outside clamping port 31 by operating the second operating lever 53, and make the release assembly 52 push the distal end portion of needle 2 through clamping port 31 to pierce the target tissue of the eye by removing the second operating lever 53 with the first stop 54.

This embodiment also provides another method of injecting ocular tissue, comprising the following steps.
Step 1, the length of needle 2 outside the clamping port 31 at the distal end of the sleeve 3 is adjusted by the adjusting release member 5 of the ocular injection device of this embodiment;
Step 2, measure the distance with ophthalmic calipers to confirm the injection site;
Step 3, clamping port 31 of the ocular injection device is placed perpendicular to the conjunctiva of the injection site;
Step 4, force is applied to the conjunctival tissue at the injection site by syringe 1 of the ocular injection device and sleeve 3 cooperatively, so that clamping port 31 is tightly attached to and clamps the conjunctival tissue to make the clamped conjunctival tissue projects toward sleeve 3;
Step 5, first stop 54 is removed using second operating lever 53 to release the release assembly 52, actuating the distal end portion of needle 2 to be inserted into the ocular target tissue from the raised conjunctival tissue through clamping port 31.
Step 6, drug is administered by injection to reach the administration site.

As shown by the results of the suprachoroidal space injection performed in accordance with the ocular injection device of the present embodiment and the ocular injection method thereof, the distal end of needle 2 of the ocular injection device of the present embodiment forms an arched structure with the projection formed by the ocular tissues, so that needle 2 of the ocular injection device can pierce the top of the projection of the ocular tissues to reach the target tissues of the ocular region, which makes it simpler to perform the injection at the identified injection site.

In industrial manufacture of the ocular injection device of this embodiment, release assembly 52 is adjusted for a stroke length that is the distance from the distal end of needle 2 to clamping port 31 when first stop 54 restricts the release thrust of release assembly 52.

### Embodiment 9

The ocular injection device of this embodiment is shown in FIGS. 17-18 and includes a thrust assembly 6. The thrust assembly 6 is configured to generate a constant thrust force on a proximal portion of the pushrod 12 to push the distal end of the pushrod 12 within the drug chamber 11 toward the needle 2, causing at least a portion of the drug within the drug chamber 11 to be injected into the target tissue of the eye via the injection site to reach the site of administration.

In the manufacture of the thrust assembly 6 of the ocular injection device of this embodiment, any of conventional mechanism, such as spring-loaded ball mechanisms, spring-loaded pins, cylinders, or container containing propellant may be used.

The operator may, when starting injection after completing the puncture, use thrust assembly 6 to generate a constant thrust force on the proximal portion of pushrod 12 to inject the drug into the target tissue of eye at a uniform rate, thereby preventing the operator from applying too large force and pushing the pushrod 12 too fast which may result in the destruction of the eye tissue of the site of administration due to the excessive pressure of the local injection.

The present embodiment of the injection device further comprises a second stop member 7 configured to selectively limit the movement of push rod 12 relative to the medication container. The second stop member 7 in the manufacture of the ocular injection device of this embodiment may utilize any mechanical structure commonly used in the prior art, such as snap rings, slots, pawls, and the like, that can limit the movement of pushrod 12 relative to the medication container. The operator may simply pull off the second stop member 7 to release thrust assembly 6 when performing the drug injection.

The ocular injection device and method of use provided herein can be widely used for ocular diseases and their associated conditions, including but are not limited to uveitis, glaucoma, diabetic macular edema or retinopathy, macular degeneration, retinal germ cell tumors, and genetic disorders, and are particularly suitable for delivery of drugs to localized areas in the posterior portion of the eye, such as the retinal choroidal tissue, the macula, and the optic nerve in the posterior section of the eye. The ocular injection device and method of use provided herein can also be used in gene-based therapy applications to deliver a medicament containing a therapeutic gene fragment into the suprachoroidal space to the target ocular tissue in any one or more carriers selected from DNA, RNA, or oligonucleotides.

The medication container 11 of the present invention contains a pharmaceutical solution of one or two or more pharmaceutically active substances selected from antibodies, antiviral agents, chemotherapeutic agents, analgesics, anesthetics, aptamers, antihistamines, anti-inflammatory agents, and antitumor agents.

The various embodiments of the present invention are described in a progressive manner, with each embodiment focusing on the differences from other embodiments, and the same or similar parts between the embodiments may be cross-referenced. Since the device disclosed in the embodiments corresponds to the method disclosed in the embodiments, it is only briefly described, and the relevant contents can be found in paragraphs illustrating the methods.

### Embodiment 10

The ocular injection assembly, as shown in FIGS. 19 to 21, comprises a sleeve 3 and a needle 2 disposed within the sleeve 3. As shown in FIG. 22 and FIG. 23, the sleeve 3 is provided with a clamping port 31 at the end of the sleeve 3. A tip portion of the needle 2 may pass through the clamping port 31 in use.

Referring to FIG. 23, the clamping port 31 has an annular end face 33, i.e., the end face of the sleeve 3 is annular in shape and includes an inner ring curve and an outer ring curve which are smooth curves and spaced. The space surrounding the inner ring curve is the clamping port. The portion between the inner ring curve and the outer ring curve is the sleeve wall. The clamping port 31 is circular in this embodiment. The annular end face 33 is shown as an annular end face in FIG. 24. In other embodiments, an elliptical annular end face as shown in FIG. 25 or a polygonal annular end face as shown in FIG. 26 may be used. Different shapes of clamping port such as round, oval, hexagonal, octagonal, square, or irregular can be used to enable the clamping port to press against the ocular tissues. The circular end surface 33 is not only more conducive to avoiding damage to the conjunctiva, but also more conducive to recovery of the ocular tissues during the injection process.

The minimum inner diameter of the clamping port 31 is 0.5mm-10mm, preferably 1mm-6mm, more preferably 1mm-3mm.

Returning to FIG. 23, the sleeve 3 ends in a constriction section 32. A clamping port 31 is located at the end of the constriction section 32. The cross-section of the constriction section 33 gradually increases in a direction away from the clamping port 31.

The length of the edge surface of the tip portion of the needle 2 is less than 1100 micrometers, preferably less than 900 micrometers, further preferably less than 700 micrometers, more preferably ≤ 550 micrometers, and most preferably 250-550 micrometers. The needle structure is referred to FIGS. 7A and 7B.

When needle 2 is mated with sleeve 3, the distance d labeled in FIG. 23 is a length of 500-2000 micrometers of the tip portion of needle 2 outside the clamping port 31. The distance from the tip of needle 2 to the annular end surface 33 is the length of the tip portion of the needle 2 outside the clamping port 31. In this embodiment annular end face 33 is planar and needle 2 is perpendicular to annular end face 33. That is, the axial direction of needle 2 is perpendicular to the plane where annular end face 33 is located.

When clamping port 31 of sleeve 3 contacts and presses on the eye tissue, clamping port 31 is sealed. As shown in FIG. 27, the ocular tissue forms a bulge 200 towards the inside of the sleeve. As shown in FIG. 28, when clamping port 31 is sealed, chamber 34 within sleeve 3 forms a hermetically sealed chamber 34. In the event of reflux, the refluxed medicament is stowed into the hermetically sealed chamber 34. When separating clamping port 31 from the ocular tissue, the medicament is less likely to leak out of the hermetically sealed chamber 34, preventing the medicament from dispersing to the ocular tissue.

Sleeve 3 is provided with a canal, and needle 2 is movably connected to the canal. Needle 2 is communicated to sleeve 3 through the canal, which facilitates the needle to maintain a position relative to the clamping port of the sleeve when the needle pierces to reach the injection point.

Returning to FIG. 22, when needle 2 is connected to sleeve 3, needle 2 has a needle hub 21 attached to needle 2 at an opposite end located at the tip portion. Needle hub 21 includes a guide tube 211 at the front end. Needle 2 is secured within guide tube 211 and extends into needle hub 21. Sleeve 3 is assembled to guide tube 211 so that sleeve 3 is fixed with respect to needle 2.

### Embodiment 11

Based on embodiment 10, in this embodiment an adjustment assembly is provided between sleeve 3 and needle 2 for adjusting the size of the length of the tip portion of the needle 2 outside the portion 31 of the clamping port. A needle hub 21 is attached to the needle 2 at an opposite end located at the tip portion, coupled to the sleeve 3 by the adjustment assembly. The adjustment assembly comprises an externally threaded section 102 provided on the needle hub 21 and an internally threaded section 101 provided on the corresponding sleeve 3. The externally threaded section 102 and the internally threaded section 101 are matingly coupled to each other. Through the mating of the externally threaded section 102 and the internally threaded section 101, the needle hub 21 and the sleeve 3 are rotated relative to each other to realize the adjustment of the length of the needle. As shown in FIG. 29, the needle hub 21 includes a guide tube 211 provided with the externally threaded section 102. The guide tube 211 is connected to the front end of the needle hub 21 and is connected to the needle for enhancing the strength of the needle to avoid bending or shaking thereof during the puncturing process. As shown in FIG. 30, sleeve 3 is provided with an internally threaded section 101. An adjustment assembly is provided between the needle hub 21 and the sleeve 3 such that adjustment of the length of needle 2 is less likely to be affected by other components such as the syringe. The thread pitch is 50-200 micrometers, more preferably 50-150 micrometers.

Another option is shown in FIG. 31. The side wall of sleeve 3 is provided with an observation window 35 of transparent material, or the sleeve is a transparent material member. The provision of the observation window 35 or the use of the transparent material sleeve facilitates the observation of the medicament reflux to quickly determine whether the injection is successful. The observation window 35 or the transparent sleeve 3 is provided with a volume scale line 351 along the axial direction of the sleeve 3 for real-time observation of the medicament reflux. When the warning limit is exceeded, it is convenient to remind the operator of the injection failure and thus stop the injection.

The adjustment assembly is provided to facilitate the adjustment of the length of the tip of needle 2 outside clamping port 31 and to control the depth of puncture, which is applicable to different patients, or to puncture different locations of the eye. For example, when a patient has abnormal sclera thickness, it may be applied to the eye wall tissue.

### Embodiment 12

This embodiment adds an adjustment assembly to embodiment 10.

As shown in FIG. 32, a needle hub 21 is connected to the rear end of the sleeve 3, and an adjustment assembly is provided therein. The adjustment assembly includes a telescoping rod assembly 42 and a drive mechanism 41. the telescoping rod assembly 42 includes an outer tube 421 and an inner tube 422. one end of the outer tube 421 is connected to the rear end of the needle hub 21 via a connecting block 423, and the other end is sheathed on the inner tube 422. The other end of the inner tube 422 is connected to the rear end of the needle 2. The outer tube 421, the inner tube 422 and the needle 2 are connected in turn. A drive mechanism 41 is connected to the inner tube 422 to drive the inner tube 422 to move axially relative to the outer tube 421 and to be able to move the needle 2. A guide tube 211 is provided outside the needle 2, and both ends of the needle 2 extend outside the guide tube 211. A front end of the needle hub 21 is disposed outside the guide tube 211. The guide tube 211 and the needle hub 21 are capable of sliding relative to each other. FIG. 33 shows that the front end of the needle 2 is incorporated into the sleeve 3 by means of an adjustment assembly.

As shown in FIG. 34, the drive mechanism 41 includes a drive housing 411, a drive gear 412, a rack guide 413, and an operating lever 414. one end of the drive housing 411 is connected to the outer tube 421, and the other end is slidably connected with the inner tube 422. The rack guide 413 is connected to the inner tube 422 and is provided in the axial direction. In this embodiment, a concave section is set in the middle of the inner tube 422, in which the rack guide 413 is directly provided. A drive gear 412 is hingedly connected to the drive housing 411 and is engaged with the rack guide 413. One end of the operating lever 414 is connected to the drive gear 412, and the other end passes through the through-hole of the drive housing 411 and the through-hole of the needle hub 21 to the outside of the needle hub 21. Turning the operating lever 412 will rotate the drive gear 412. By rotating the operating lever 414 in the rotary drive mechanism to turn the drive gear 412 which then moves the rack guide 413. The inner tube 422 moves with the rack guide 413 and drives the needle 2 in the axial direction, realizing the adjustment of the position of the needle 2.

Another embodiment is shown in FIGS. 35 and 36. The inner tube 422 includes upper and lower sections, wherein the upper section is socketed within the outer tube 421 and the lower section is connected to the guide tube 211. The upper section and the lower section are connected to each other by a rack guide 413 to facilitate setting the drive gear 412 in the middle of the drive housing 411.

A further embodiment is shown in FIG. 37. The rear end of the needle 2 passes through the inner tube 422 and the outer tube 421 in turn and extends outside the outer tube 421. The needle 2 is fixedly connected to the inner tube 422 by a guide tube 211, and the rear end of the needle 2 extends outside the inner tube 422 toward the outer tube 421 to the other end of the outer tube 421. The rear end of the needle 2 extends into the syringe in use with a syringe.

### Embodiment 13

This embodiment adds a release assembly to embodiment 10.

As shown in FIG. 38, a needle hub 21 is attached to the rear end of the sleeve 3. A release assembly 5, including a resilient member 51 and a first stop member 52, is provided between the sleeve 3 and the needle hub 21. The sleeve 3 is connected to the needle hub 21 via the resilient member 51. One end of the first stop member 52 is removably connected to the sleeve 3 and/or the other end is removably connected to the needle hub 21. When the first stop member 32 is connected to the sleeve 3 and the needle hub 21, the resilient member 51 is in a stretched state, providing tension along the axial direction. The resilient member 51 may be a reset spring, a compressed gas container or a container containing propellant. The first stop member 52 may be a tab, a slot, a ring, a slot or a pawl, etc. In this embodiment the resilient member 51 is a reset spring and the first stop member 52 is a snap ring, as shown in FIG. 40.

As shown in FIG. 38, the resilient member 51 is stretched to increase the distance between the needle hub 21 and the sleeve 3. The first stop member 52 is snapped between the needle hub 21 and the sleeve 3 to maintain the distance between the needle hub 21 and the sleeve 3. At this point the resilient member is in a stretched state and the front end of the needle 2 is recovered into the sleeve 3. The clamping port 31 is kept in contact with the eye tissue during use. After removing the first stop member 52 the needle hub 21 is moved forwardly so that the front end of the needle 2 is outside the clamping port 31, as shown in FIG. 39, and pierces into the eye tissue.

Similarly, an adjustment assembly is provided between the sleeve 3 and the needle hub 21, including a telescoping rod assembly 42 and a drive mechanism 41. The telescoping rod assembly 42 includes an outer tube 421 and an inner tube 422. One end of the outer tube 421 is connected to the front end of the needle hub 21, and the other end is sheathed on the inner tube 422. The other end of the inner tube 422 is connected to the rear end of the needle hub 21. The outer tube 421, the inner tube 422 and the needle 21 are connected in turn, or the rear end of the needle 2 passes through the inner tube 422 and the outer tube 421 in turn and extends outside the outer tube 421. the drive mechanism 41 is connected to the inner tube 422 to drive the inner tube 422 to move in an axial direction with respect to the outer tube 421 and drive the needle 21.

As shown in FIG. 34, the drive mechanism 41 includes a drive housing 411, a drive gear 412, a rack guide 413, and an operating lever 414. One end of the drive housing 411 is connected to the outer tube 421, and the other end is slidingly connected to the inner tube 422. The rack guide 413 is connected to the inner tube 422 and is provided in an axial direction. A drive gear 412 is hingedly connected within the drive housing 411 and is engaged with the rack guide 413. One end of the operating lever 414 is connected to the drive gear 412, and the other end passes through the through-hole of the drive housing 411 and the through-hole of the needle hub 21 to the outside of the needle hub 21. Turning the operating lever 414 will rotate the drive gear 412.

In yet another embodiment, the operating lever 414 is detachably connected to the drive gear 412. The first stop 52 may be driven when the operating lever 414 is separated from the needle hub 21 to separate the first stop member 52 from the sleeve 3 and/or the needle hub 21. By providing the operating lever 414 outside the needle hub 21, after completion of adjusting the length of the needle 2 using the operating lever 414, the operating lever 414 can be removed by pulling out it for easy observation. Moreover, the operation lever 414 drives the first stop member 52 to separate from the sleeve 3 and the needle hub 21, which facilitates one-handed operation by the operator. In this embodiment, the structure of the adjustment assembly 4 may be the same as the structure of the adjustment assembly in the previous embodiment, or the adjustment assembly may be provided outside the needle hub and in the middle of the resilient member 5.

### Embodiment 14

The ocular injection device is shown in FIG. 41 and includes a syringe 1 and an ocular injection assembly of any of embodiments 10-13 mounted on the syringe.

As shown in FIG. 42, the syringe 1 includes a medication container 11, a pushrod 12. the pushrod 12 is slidable within the medication container 11. The medication container 11 is used to store the medication and is matingly connected to the injection assembly 2. The injection assembly also includes a needle protection cap 8 that fits over the needle 2.

### Embodiment 15

This embodiment is based on embodiment 14, and a thrust assembly 6 is provided between the end of the pushrod 12 and the medication container 11 for generating a constant thrust force on the pushrod.

The thrust assembly 6 is a spring-loaded ball mechanism, a spring-loaded pin, a cylinder, or a container storing a propellant, which generates a constant thrust with a threshold value of no more than 6 N. The thrust assembly 6 generates a constant thrust in such a way that it is not able to propel the thrust assembly when the thrust applied to the thrust assembly is less than a threshold value, and the thrust assembly is capable of counteracting the thrust to maintain the thrust at the threshold when the thrust is greater than the threshold value. For example, when the threshold value is 5N, the thrust assembly cannot be pushed using a force of 4N, and when the thrust force is 7N, the thrust assembly is able to maintain a thrust force of 6N.

The injection device further comprises a second stop member 7 provided between the pushrod 12 and the medication container 11 for limiting the size of the pushing stroke of the pushrod. The second stop member 7 may be a tab, a slot, a ring, a slot or a pawl.

### Embodiment 16

A method of using an ocular injection device, using the ocular injection device of embodiment 14 or 15, contacting and pressing the clamping port 31 of the sleeve 3 against the ocular tissue at the injection site so that the ocular tissue at the injection site forms a bulge 200 into the sleeve 3.

After the sleeve clamping port 31 is contacted and pressed against the ocular tissue at the injection site to form a bulge 200 of the ocular tissue at the injection site into the sleeve, the tip portion of the needle 2 is then made to puncture the conjunctival tissue and scleral tissue at the injection site to allow the distal end of the needle to reach the target tissue of the eye at the injection site.

The length of the tip portion of the needle exposed to the clamping port is adjusted according to the thickness of the ocular tissue at the injection site before the sleeve clamping port 31 contacts the ocular tissue at the press injection site. The adjustment is performed using the adjustment assembly in the injection assembly described above.

Alternatively, one side of the sleeve clamping port 31 is first brought into contact with the ocular surface, then the sleeve clamping port 31 is pivoted using that contact part as a pivot point to allow the needle to pierce the ocular tissue at the injection site, and is further pivoted so that the other side of the clamping port 31 is in contact with the ocular surface, the ocular tissue forms a bulge into the sleeve 3. The ocular surface may be a conjunctival surface or other ocular tissue.

Ocular tissue thickness at the injection site was obtained by testing with one or more of optical coherence tomography (OCT), enhanced deep imaging with OCT (EDI-OCT), swept-frequency OCT (SS-OCT), or ultrasound biomicroscopy (UBM).

After the distal end of the needle 2 reaches the target tissue of the eye at the injection site, the pushrod 12 is pushed to inject at least a portion of the substance in the medication container 11 into the target tissue of the eye via the needle 2.

While pushing the pushrod 12 to deliver at least a portion of the substance in the medication container 11 to the target tissue of the eye via the distal end of the needle 2, a return volume of the refluxed drug solution in the sleeve 3 is observed. The observation can be made using an observation window or a transparent sleeve in the injection device, and the volume of the refluxed medicament is recorded with reference to a volume scale.

### Test example 1

Experiment No. 1-1. The ocular injection assembly and syringe of embodiment 10 were used to form an injection device, and a socket needle with an effective length of 700µm and a blade length of 500±50µm was set according to the conventional thickness of the New Zealand rabbit scleral tissues and choroid, and the socket clamping port 31 was round with an inner diameter of 1.5mm.

The effective length, i.e., the length of the tip portion of needle 2 outside clamping port 31, is 700µm. That is, the distance from the tip portion of needle 2 to the annular end surface is 700µm. Needle 2 is substantially perpendicular to the annular end surface.

Experimental method: after pentobarbital anesthesia, ocular surface anesthesia was performed using proparacaine hydrochloride eye drops, and waited for 1-2 minutes after the eye drops, then suprachoroidal space injection was performed using a homemade socket needle.
Test animals: Healthy New Zealand rabbits, 1.8-2.2 kg;
Injection frequency: single-site injection in the right eye;
Injection reagent: 0.2% ICG, 100 µl;
The method of suprachoroidal space injection comprises the following steps.

A first step is to measure the distance with ophthalmic calipers to confirm the injection site.

In the second step, the injection reagent is drawn and a force is applied to the side of the clamping port 31 via the syringe, so that the side of the clamping port 31 is tightly pressed against the ocular tissues at the injection site and forms a fulcrum on the ocular surface of the injection site.

In the third step, the injection device is flipped around the pivot point to the other side of the clamping port 31 so that the distal end of the needle 2 pierces the ocular tissue at the injection site.

In the fourth step, the other side of the clamping port 31 is attached closely to the ocular surface of the injection site by continuing to flip around the pivot point, so that the clamping port 31 grips the ocular tissue to form a bulge 200 into the sleeve 3. The distal end of the needle 2 is pressed vertically into the sclera to reach the target tissue of the eye at the injection site.

In the fifth step, the drug is injected to reach the site of administration.

The injection conditions such as reflux, ocular surface spread, and subconjunctival residue, spread, hemorrhage, or congestion were recorded during the injection process. Rabbits were executed, and the eyeballs were dissected to prepare flat samples of fundus tissue. Photographs were taken to record the distribution of ICG solution in the suprachoroidal space.

Test result: It is observed that there was no reflux and no subconjunctival residue caused by ICG injections, and the results of fundus tissue sections showed that all the fundus tissues were transfected with ICG, and the stained fundus tissues were light green in color. The fundus tissue sections are shown in FIG. 44.

### Test example 2

On the basis of Test example 1, other conditions were kept constant, and the inner diameter of the sleeve clamping port was adjusted, thereby investigating the effect of the inner diameter of sleeve 3 on the reflux of the drug solution. The inner diameters of sleeve 3 were 0.25 mm (Experiment No. 1-2), 0.5 mm (Experiment No. 1-3), 1.0 mm (Experiment No. 1-4), 2.0 mm (Experiment No. 1-5), 2.5 mm (Experiment No. 1-6), 3.0 mm (Experiment No. 1-7), 5.0 mm (Experiment No. 1-8) and 10.0 mm (Experiment No. 1-9), respectively.

Test example 1 and Test example 2 are summarized below:

**Table 1 Effect of sleeves with different inner diameters on return flow**

| test serial number | Sleeve inner diameter (mm) | Experimental Phenomena (right eye) | Experimental Phenomena (Tissue slices) |
|---|---|---|---|
| 1-1 | 1.5 | No reflux, and no ocular surface and subconjunctival spreading | All transfected with ICG, light green |
| 1-2 | 0.25 | Significant reflux, significant ocular surface and subconjunctival spreading | Severe reflux, failed injections, rabbits not executed for preparing fundus sections |
| 1-3 | 0.5 | Significant reflux, significant ocular surface and subconjunctival spreading | Severe reflux, failed injections, rabbits not executed for preparing fundus sections |
| 1-4 | 1.0 | No significant reflux, and no ocular surface and subconjunctival spreading | About 3/4 of the area stained, green |
| 1-5 | 2.0 | No significant reflux, no ocular surface spreading and trace residue in the subconjunctiva but not spreading | Over 3/4 of the area stained, dark green |
| 1-6 | 2.5 | No significant reflux, no ocular surface spreading and trace residue in the subconjunctiva but not spreading | About 2/3 of the area stained, dark green |
| 1-7 | 3.0 | No significant reflux, no ocular surface spreading and small subconjunctival residue, slight bulging but not spreading | About 3/5 of the area stained, dark green color |
| 1-8 | 5.0 | No reflux, no ocular surface spread and no visible subconjunctival residue | Fundus tissue unstained, vitreous stained |
| 1-9 | 10.0 | No reflux, no ocular surface spread and no visible subconjunctival residue | Fundus tissue unstained, vitreous stained |

The test results showed no significant reflux or ocular surface and subconjunctival spreading at an internal diameter of 1-3 mm. Tissue section conditions showed a stained area between 3/5 and 3/4 of the area, and the injection was successful.

### Test example 3

Unlike Test example 1, the injection reagent was AAV8-EGFP recombinant adeno-associated virus, in which the target gene was EGFP, dosage: 1.02E+11vg/eye, single-site injection in the left eye.

The injection conditions such as reflux, ocular surface spread, and subconjunctival residue, spread, hemorrhage, or congestion were recorded during the injection process. Immediately after injection, OCT was performed on the injected eye to observe whether the injection site was the suprachoroidal space, whether it caused damage to the surrounding tissues, and to assess the extent of drug diffusion in the suprachoroidal space. After injection, the animals were continued to be fed for observation and were examined by autofluorescence at 28 days. Eyes were isolated after euthanizing the animals, and frozen sections and staining were performed to examine AAV-EGFP transduction.

Test results: It is observed that there was no reflux or ocular surface spreading caused by the drug injection, and there was no residue, hemorrhage, or congestion in the conjunctiva. The OCT scan results are shown in FIG. 45, with no scleral puncture openings. The choroidal detachment was obvious on OCT, and there was no retinal detachment, bulge, or lacrimation, which indicated that the injection depth was appropriate without penetrating the choroid or the retina. Autofluorescence detection was shown in FIG. 46, with fluorescent dots distributed throughout the fundus on the upper and lower left and right sides, and the optic disc was obvious on the upper and lower sides. Frozen retinal tissue sections are shown in FIG. 47, and the injected reagent was clearly expressed in retinal epithelial cells and photoreceptor cells. The above test results showed that the injection was successful.

Note: RGC, ganglion cells; INL, inner nuclear layer of the retina; ONL, outer nuclear layer of the retina; RPE, retinal pigment epithelial cells.

### Test example 4

The ocular injection assembly and syringe of Embodiment 1 were used to form an injection device, and a socket needle with an effective length of 700µm and a blade length of 500±50µm was customized according to the conventional thickness of the scleral tissues and choroid of rhesus monkeys, with a sleeve 3 having an inner diameter of 1.5 mm.

The effective length, i.e., the length of the tip portion of the needle 2 outside the portion of the clamping port is 700µm. That is, the distance from the tip portion of the needle 2 to the annular end surface is 700µm; and the needle 2 is substantially perpendicular to the annular end surface.

Experimental method: After pentobarbital anesthesia, ocular surface anesthesia was performed using proparacaine hydrochloride eye drops, and waited for 1-2 minutes after the drops, suprachoroidal space injection was performed using a homemade socket needle.
Test animals: rhesus monkeys;
Frequency of injections: single-site injections in both eyes;
Injection reagent: 0.2% ICG, 100µl;
Injection method: same as in Test example 1;

The injection conditions such as reflux, ocular surface spread, and subconjunctival residue, spread, hemorrhage, or congestion were recorded during the injection process. Immediately after injection, ICGA contrast and OCT were performed on the injected eye to observe whether the injection site was the suprachoroidal space and whether damage is caused to the surrounding tissues, and to assess the extent of drug diffusion in the suprachoroidal space.

Test results: It is observed from the injection results that there was a small amount of reflux in the right and left eyes, the reflux fluid was adsorbed by the sleeve, no ocular surface diffusion was seen, and no subconjunctival residue, diffusion, hemorrhage, or congestion was seen. ICGA contrast results as shown in Fig. 48 showed that striated hyper-fluorescence was seen in the right and left eyes, localized obvious fluorescence at the injection point was visible, no intravascular hyper-fluorescence was seen, no diffuse lamellar fluorescence was seen, and fluorescence diffusion was seen up to supratemporal level. OCT scanning results were shown in FIG. 48, with extra choroidal dark areas visible in both the right and left eyes, shallow choroidal detachment, and no retinal detachment, bulge, or lacunae. The above test results showed that the injection was successful.

### Test example 5

The difference between this Test example and Test example 4 is that the socket needle with an effective length of 800µm and a blade length of 500 ± 50µm was customized according to the conventional thickness of the scleral tissue and choroid of rhesus monkeys, and the inner diameter of the sleeve 3 was 1.5 mm.

Test results: it is observed from the injection results that a small amount of reflux present in the left eye and a trace amount of reflux in the right eye, but the reflux fluid in both eyes was adsorbed by the sleeve, and no ocular surface diffusion, subconjunctival residue, diffusion, hemorrhage or congestion was seen in either eye. The results of trans-ICGA contrast are shown in FIG. 49, with streaked hyper-fluorescence in both the right and left eyes, visible localized obvious fluorescence at the injection site, no intravascular hyper-fluorescence, no diffuse flakiness fluorescence, and fluorescence can be seen at the macula or supratemporal. The results of the OCT scans are shown in FIG. 49, with extra-choroidal dark areas and superficial choroidal detachment in both the right and left eyes; the retina of the left eye was slightly elevated, and the right eye did not have retinal detachments, elevations, or lacunae. The test results showed that the injection was successful.

### Test example 6

The difference between this Test example and Test example 4 is that the scleral thickness was examined. Injection device used is a socket needle with a blade length of 500 ± 50µm and including a threaded adjusting member to adjust the effective length at 2,000µm, and a single-site injection in the right eye was performed.

### The injection method is as follows:

In a first step, the operator detected the thicknesses of retinal, choroidal, and scleral in the injection region of the right eye of rhesus monkeys using any one of optical coherence tomography, OCT-enhanced deep imaging, swept-frequency OCT, or ultrasound biomicroscopy, and used the adjusting component 4 of the ocular injection device to adjust the length of the needle 2 outside the clamping port 31 of the sleeve 3 in accordance with the detected thicknesses, wherein the adjusted length of the right eye was 650µm.

In a second step, measure the distance with ophthalmic calipers to confirm the injection site.

In a third step, clamping port 31 of the ocular injection device is placed perpendicular to the conjunctiva of the injection site.

In a fourth step, a force is applied to the conjunctival tissue at the injection site through the syringe 1 of the ocular injection device and the sleeve 3 in conjunction, so that the clamping port 31 was close-fitting and gripped on the conjunctival tissue, so that the gripped conjunctival tissue is projected toward the sleeve 3. The distal end of the needle 2 is vertically pierced into the scleral tissue to reach the target tissue of the eye at the injection site.

In a fifth step, the drug is administered by injection so that the drug reaches the site of administration.

The injection conditions such as reflux, ocular surface spread, and subconjunctival residue, spread, hemorrhage, or congestion were recorded during the injection process. Immediately after injection, ICGA contrast and OCT were performed on the injected eye to observe whether the injection site was the suprachoroidal space and whether damage is caused to the surrounding tissues, and to assess the extent of drug diffusion in the suprachoroidal space.

Test results: it is observed from the injection result that no reflux, ocular surface diffusion, and subconjunctival residual, diffuse hemorrhage or congestion was seen. ICGA contrast was shown in FIG. 50, in which streaky hyper-fluorescence was visible, localized significant fluorescence at the injection site, no intravascular hyper-fluorescence, and no diffuse lamellar fluorescence, which was fluorescent up to the macular area or supratemporal level. OCT scans, as shown in Fig. 50, did not show any scleral puncture openings, and the OCT visualized choroidal detachment was evident, and no retinal detachment, bulge, or fissure was seen. The test results showed that the injection was successful.

### Test example 7

The difference between this Test example and Test example 4 is that the scleral thickness was examined, and the injection device used is a socket needle with a needle 2 having a blade length of 400±50µm and including a threaded adjustment member to adjust the effective length at 2000µm, and that a single-site injection in the left eye was performed.

The injection method was the same as in Test example 6, in which the left eye was adjusted to a length of 750µm.

The injection conditions such as reflux, ocular surface spread, and subconjunctival residue, spread, hemorrhage, or congestion were recorded during the injection process. Immediately after injection, OCT was performed on the injected eye to observe whether the injection site was the suprachoroidal space and whether damage is caused to the surrounding tissues, and to assess the extent of drug diffusion in the suprachoroidal space. The animals were continued to be kept for observation for 28 days after injection. After euthanizing the animals, the eyeballs were separated, and frozen sections and staining were performed to examine AAV-EGFP transduction.

Test results: it is observed from the injection results that no reflux, ocular surface diffusion as well as subconjunctival residual, diffuse hemorrhage or congestion is visible. OCT scanning results were shown in FIG. 51, and no scleral puncture opening was seen. OCT showed obvious choroidal detachment, no retinal detachment, bulge or lacunae, indicating that the injection depth was appropriate and did not penetrate the choroid and retina. Frozen retinal tissue sections are shown in FIG. 52, which shows that after injection through the suprachoroidal space, AAV-EGFP mainly transduced RPE cells and photoreceptor cells. The test results showed that the injection was successful.

In the figure, RPE is retinal pigment epithelium, IS/OS is retinal photoreceptor inner and outer ganglion connections, INL is inner nuclear layer of the retina, ONL is outer nuclear layer of the retina, and RGC is ganglion cells.

### Test example 8

This Test example differs from Test example 4 in that the scleral thickness was examined, and using an injection device which is a socket needle containing a threaded adjusting member for adjusting the effective length of a needle blade length of 300±50µm, with an adjustable effective length of 2000pm, wherein the injection device was provided with a thrust assembly and a single-point injection for the right eye.

### The injection method is as follows:

In a first step, the operator detected the thickness of the conjunctiva to the choroid in the injection area of the rhesus monkey using optical coherence tomography, OCT-enhanced deep imaging, swept OCT, or ultrasound biomicroscopy, and adjusted the length of the needle exposing the clamping port of the sleeve according to the detected conjunctiva to choroid thickness via the adjustment component of the ocular injection device, in which the adjustment length was 850µm for the right eye.

In a second step, measure the distance with ophthalmic calipers to confirm the injection site.

In a third step, the clamping port 31 of the ocular injection device is placed perpendicular to the ocular surface at the injection site.

In a fourth step, the operator applied a force to the ocular tissue at the injection site via the thrust assembly 6 via the syringe of the ocular injection device and the sleeve 3 in conjunction, such that the clamping port 31 abuts and grips the ocular tissue to cause the ocular tissue gripped to form a project 200 towards the sleeve 3, so that the distal end of the needle 2 is pressed vertically from the projecting ocular surface tissue into the sclera through the clamping port of the sleeve 3 to reach the target tissue of the eye at the injection site.

In a fifth step, the operator opens the second stop member 7, presses the distal end of the pushrod 12, and injects the medication through the constant thrust created by the thrust assembly on the proximal portion of the pushrod 12, so that the medication reaches the site of administration.

The injection conditions such as reflux, ocular surface spread, and subconjunctival residue, spread, hemorrhage, or congestion were recorded during the injection process. Immediately after injection, OCT was performed on the injected eyes to observe whether the injection site was the suprachoroidal space and whether damage is caused to the surrounding tissues, and to assess the extent of drug diffusion in the suprachoroidal space. After injection, the animals were continued to be kept for observation and were examined by autofluorescence at 28 days. The eyes were isolated after euthanizing the animals, and frozen sections and staining were performed to examine AAV-EGFP transduction.

Test results: Observation of the injection results showed no reflux, ocular surface diffusion, and subconjunctival residual, diffuse hemorrhage or congestion. OCT scan results were shown in FIG. 53, and no choroidal or retinal detachment, bulge, or lacunae was seen, suggesting that the injection depth was appropriate, and that it did not penetrate the choroid or the retina. Frozen retinal tissue sections are shown in FIG. 54, after injection through the suprachoroidal space, AAV-EGFP mainly transduced RPE cells and photoreceptor cells. The test results showed that the injection was successful.

### Test example 9

This Test example differs from Test example 4 in that the injection dose was 100 microliters per dose per eye.

Monocular injections were performed according to the injection method of Test example 4, in which three monkeys were injected with ICG, three monkeys were injected with buffer, and the remaining nine monkeys were injected with AAV8-containing drugs.

The injection conditions such as reflux, ocular surface spread, and subconjunctival residue, spread, hemorrhage, or congestion were recorded during the injection process.

Test results: no reflux, ocular surface spread, or subconjunctival residual, spreading hemorrhage or congestion was seen in any of the 36 eyes during the injection. The test results showed that the injection was successful.

### Comparative example 1

### Open conjunctival injection method for suprachoroidal space injection

The Comparative example differs from Test example 1 in that a common syringe was used for injection.

### Syringes: BD syringe, 34G needle; WPI 34G blunt needle

Test method: After pentobarbital anesthesia, proparacaine hydrochloride eye drops were used for ocular surface anesthesia, and waited for 1 min-2 min after the eye drops, the suprachoroidal space was injected using the open conjunctival injection method. Specific procedures: the conjunctiva was first cut open using conjunctival scissors to completely expose the sclera at the injection site, then the sclera was punctured using a BD syringe needle 34G, and finally the suprachoroidal space was injected using a WPI 34G blunt needle.

The injection conditions such as reflux, ocular surface spread, and subconjunctival residue, spread, hemorrhage, or congestion were recorded during the injection process. After execution of the rabbits, their eyeballs were dissected and flat samples of the fundus tissue were prepared, and photographs were taken to record the distribution of the ICG solution in the suprachoroidal space.

Test results: it is observed that severe reflux and subconjunctival residue of the injected ICG, severe ocular surface and subconjunctival spreading, and conjunctival hemorrhage occurred. Fundus tissue section results showed no staining of the fundus tissue. The test results showed that the injection was unsuccessful.

### Comparative example 2

### Hub body microneedle was used for suprachoroidal space injection (without sleeve)

Hub body microneedle used was the one with an effective length for suprachoroidal space being set as 700 µm and a blade length being set as 450 µm based on the scleral tissue and the regular thickness of the choroid in New Zealand rabbits.

Test methods: after pentobarbital anesthesia, ocular surface anesthesia was performed using proparacaine hydrochloride eye drops, waited for 1-2 minutes after the eye drops, and the sclera was punctured vertically using a homemade hub-body microneedle and suprachoroidal space injection was performed.
Test animals: healthy New Zealand rabbits, 1.8-2.2 kg
Injection frequency: single-site injection in the right eye
Injection reagent: 0.2% ICG, 100 µl
The injection conditions such as reflux, ocular surface spread, and subconjunctival residue, spread, hemorrhage, or congestion were recorded during the injection process. After execution of the rabbits, the eyeballs were dissected and flat samples of the fundus tissue were prepared, and photographs were taken to record the distribution of the ICG solution in the suprachoroidal space.

Test results: it is observed that ICG injection reflux and subconjunctival residue were obvious, ocular surface and subconjunctival diffusion were obvious, and a small amount of congestion existed in the subconjunctiva. As shown in FIG. 55, the results of the fundus tissue section showed that the ICG-stained area of the fundus tissue was less than 2/3, and the stained fundus tissue was light green. The test results showed that the injection was unsuccessful.

The various embodiments of the present invention are described in a progressive manner, with each embodiment focusing on the differences from other embodiments, and the same or similar parts between the embodiments may be cross-referenced. Since the device disclosed in the embodiments corresponds to the method disclosed in the embodiments, it is only briefly described, and the relevant contents can be found in paragraphs illustrating the methods.

Although the devices and methods of the present invention are described for delivering a drug in the suprachoroidal space, in other embodiments, the devices and methods of the disclosure may be adapted to deliver any suitable therapeutic substance to any portion of the eye, such as the cornea, the conjunctiva, the retinal region, or the vitreous. In other embodiments, either of the disclosed devices and methods may be suitable for delivering any suitable therapeutic substance to any desired target tissue of the eye.

Specific components arranged in particular orientations or positions as indicated above in conjunction with the schematic drawings and/or embodiments may be adapted. Similarly, the order of particular methods and/or steps of the disclosure may be adapted. Although embodiments have been specifically shown and described, it should be understood that various changes in form and detail may be made.

An ocular injection assembly, an injection device and a method of use provided by the present invention are described in detail above. Specific embodiments have been applied herein to illustrate the principles and embodiments of the present invention. The above illustrations of the embodiments are only for helping understanding of the method of the present invention and its core ideas. It should be noted that, a number of improvements and modifications can be made to the present invention for a person of ordinary skilled in the art without departing from the principles of the present invention, and these improvements and modifications also fall within the scope of protection of the claims of the present invention.

## Claims

1. An ocular injection assembly comprising a sleeve and a needle, wherein the needle can be set within the sleeve, a clamping port is provided at the end of the sleeve, and the tip portion of the needle can pass through the clamping port, and wherein when the ocular injection assembly is used, the clamping port is contacted with and pressed against ocular tissues at the site of the injection to cause the ocular tissues at the site of the injection to protrude into the sleeve.

2. The ocular injection assembly according to claim 1, wherein the clamping port has an annular end surface.

3. The ocular injection assembly according to claim 1, wherein the clamping port has a minimum inner diameter of 1 mm-3 mm.

4. The ocular injection assembly according to claim 2, wherein the needle is perpendicular to the annular end surface.

5. The ocular injection assembly according to claim 1, wherein the clamping port is of circular, oval or polygonal shape.

6. The ocular injection assembly according to claim 1, wherein the end of the sleeve is a constricted section and the clamping port is located at the end of the constricted section; the constricted section has a cross-sectional size that is progressively larger in a direction away from the clamping port.

7. The ocular injection assembly according to claim 1, wherein the side wall of the sleeve is provided with an observation window of transparent material, or the sleeve is a transparent material member, preferably the observation window or the transparent sleeve is provided with a volume scale along the axial direction of the sleeve.

8. The ocular injection assembly according to claim 1, wherein the needle tip portion has a blade length less than or equal to 1100 micrometers, preferably less than or equal to 900 micrometers, further preferably less than or equal to 700 micrometers, further preferably less than or equal to 550 micrometers, further preferably of 250-550 micrometers, more preferably of 400-550 micrometers.

9. The ocular injection assembly according to claim 1, wherein the needle, when mated with the sleeve, extends with its tip portion outside the clamping port for 500-2000 micrometers; preferably the length of the tip portion of the needle outside the clamping port facilitates delivery of the medicament into the suprachoroidal space.

10. The ocular injection assembly according to claim 1, wherein the sleeve is provided with a canal and the needle is movably attached within the canal.

11. The ocular injection assembly according to any one of claims 1-10, wherein a needle hub is attached to the needle at an end opposite to the tip portion, and the sleeve and the needle hub are provided with an adjusting assembly for adjusting the length of the tip portion outside the clamping port.

12. The ocular injection assembly according to claim 11, wherein the needle hub is coupled to the sleeve via the adjustment assembly.

13. The ocular injection assembly according to claim 12, wherein the adjusting assembly comprises a male threaded section provided on the needle hub, and a female threaded section provided on the corresponding sleeve, the male threaded section and the female threaded section being cooperatively connected to each other.

14. The ocular injection assembly according to claim 13, wherein the needle hub comprises a guide tube provided with the externally threaded section.

15. The ocular injection assembly according to any of claims 1-10, wherein a needle hub is connected to the rear end of the sleeve; the needle hub is provided with an adjustment assembly comprising a telescopic rod assembly and a drive mechanism;
the telescoping rod assembly includes an outer tube and an inner tube, one end of the outer tube is connected to the rear end of the needle hub and the other end is sheathed on the inner tube, and the other end of the inner tube is connected to the rear end of the needle; and the outer tube, the inner tube, and the needle are sequentially connected, or, the rear end of the needle passes sequentially through the inner and the outer tubes and outside the outer tube; and
the drive mechanism is connected to the inner tube to drive the inner tube axially relative to the outer tube, and the inner tube is capable of moving the needle.

16. The ocular injection assembly according to claim 15, wherein the needle is provided with a guide tube over the outer sleeve of the needle, the ends of the needle are exposed to the guide tube, the front end of the needle hub is set outside the guide tube, and the guide tube and the needle hub are capable of sliding relative to each other.

17. The ocular injection assembly according to claim 16, wherein the drive mechanism comprises a drive housing, a rack and pinion guide, a drive gear and an operating lever;
one end of the drive housing is attached to the outer tube and the other end of the drive housing is slidingly connected to the inner tube;
the rack and pinion guide is connected to the inner tube axially; the drive gear is hingedly connected in the drive housing in engagement with the rack and pinion guide; and
one end of the operating lever is connected to the drive gear and the other end passes through a through-hole of the drive housing and a through-hole of the needle hub to the outside of the needle hub; the operating lever is turned to rotate the drive gear.

18. The ocular injection assembly according to any one of claims 1-10, wherein a needle hub is connected to the rear end of the sleeve, and a release assembly comprising a resilient member and a first stop member is provided between the sleeve and the needle hub; the sleeve is connected to the needle hub via the resilient member;
one end of the first stop member is detachably connected to the sleeve and/or the other end of the first stop member is detachably connected to the needle hub;
when the first stop member is connected to the sleeve and the needle hub, the resilient member is in a tensile state, capable of providing tension along the axial axis.

19. The ocular injection assembly according to claim 18, wherein the resilient member is a reset spring, a compressed gas container, or a container comprising a propellant; and the first stop is a tab, a slot, a ring, a slot, or a detent.

20. The ocular injection assembly according to claim 18, wherein an adjustment assembly comprising a telescoping rod assembly and a drive mechanism is provided between the sleeve and the needle hub;
the telescoping rod assembly comprises an outer tube and an inner tube, one end of the outer tube is connected to the front end of the needle hub and the other end is sheathed on the inner tube, the other end of the inner tube is connected to the rear end of the needle;
the outer tube, the inner tube and the needle are sequentially connected, or the rear end of the needle passes sequentially through the inner tube and the outer tube and outside the outer tube;
the drive mechanism is connected to the inner tube and is capable of driving the inner tube in an axial direction relative to the outer tube, the inner tube being capable of moving the needle.

21. The ocular injection assembly according to claim 20, wherein the drive mechanism comprises a drive housing, a rack guide, a drive gear and an operating lever;
the drive housing is attached to the outer tube at one end and slidingly connected to the inner tube at the other end;
the rack guide is connected to the inner tube axially; the drive gear is hingedly connected within the drive housing and is engaged with the rack guide;
One end of the operating lever is connected to the drive gear and the other end passes through a through-hole in the drive housing and a through-hole in the needle hub to the outside of the needle hub; the operating lever is turned to rotate the drive gear;
the operating lever is removably connected to the drive gear; the operating lever, when being separated from the needle hub, is capable of driving the first stop to cause the first stop to be separated from the sleeve and/or the needle hub.

22. The ocular injection assembly according to any one of claims 1-21, wherein the chamber within the sleeve forms a hermetically sealed chamber when the clamping port is sealed.

23. An ocular injection device comprising a syringe, and an ocular injection assembly as claimed in any one of claims 1-22 being mountable on the syringe.

24. The ocular injection device according to claim 23, wherein the syringe comprises a medication container and a pushrod, the pushrod being capable of sliding within the medication container, the medication container being used for storing a medicinal solution, and the medication container being cooperatively connected to the needle.

25. The ocular injection device according to claim 24, wherein a thrust assembly is provided between the end of the pushrod and the medication container to apply a constant thrust at the pushrod.

26. The ocular injection device according to claim 25, wherein the thrust assembly is a spring-loaded ball mechanism, a spring-loaded pin, a cylinder, or a container containing a propellant.

27. The ocular injection device according to claim 25, wherein the thrust assembly produces a constant thrust with a threshold value of no more than 6N.

28. The ocular injection device according to claim 24, further comprising a second stop member provided between the pushrod and the medication container to limit the pushing stroke of the pushrod.

29. The ocular injection device according to claim 28, wherein the second stop is a tab, slot, ring, groove or pawl.

30. The ocular injection device according to claim 23, further comprising a needle protection cap capable of cooperating with the needle.

31. A method of using an ocular injection device as claimed in any one of claims 23-30, comprising using the ocular injection device to contact and press the clamping port of the sleeve against ocular tissue at the injection site to cause the ocular tissue at the injection site to bulge into the sleeve.

32. The method of use according to claim 31, wherein after the clamping port of the sleeve contacts and presses the ocular tissue at the injection site to form a bulge of the ocular tissue into the sleeve, the tip portion of the needle punctures the ocular tissue at the injection site to reach the distal end of the needle to the target ocular tissue at the injection site.

33. The method of use according to claim 31, wherein while the clamping port of the sleeve contacts and presses the ocular tissue at the injection site to form a bulge of ocular tissue into the sleeve, the tip portion of the needle punctures the ocular tissue at the injection site to allow the distal end of the needle to reach the target tissue of the eye at the injection site.

34. The method of use according to claim 31, wherein the length of the tip portion of the needle outside the clamping port is adjusted according to the thickness of the ocular tissue at the injection site before the clamping port of the sleeve contacts the ocular tissue at the injection site of compression.

35. The method of use according to claim 31, wherein one side of the clamping port is first brought into contact with the eye surface to become a pivot part, then the clamping port is pivoted around the pivot part so that the needle pierces the eye tissue at the injection site, and the clamping port is further pivoted so that the other side of the clamping port is brought into contact with the eye surface and the eye tissue forms a bulge into the sleeve.

36. The method of use according to claim 31, wherein the ocular tissue thickness at the injection site is obtained by detection using one or more of optical coherence tomography, OCT-enhanced deep imaging, swept-frequency OCT or ultrasound biomicroscopy.

37. The method of use according to claim 31, wherein pushing the pushrod injects at least a portion of the substance of the medication container into the target tissue of the eye via the needle.

38. An ocular injection device comprising a syringe, a needle, and a sleeve, the sleeve being provided with a clamping port at the distal end, wherein the clamping port is tightly attached to ocular tissues when injecting into the eye and can cause the ocular tissues to form a bulge towards the inner end of the sleeve.

39. The ocular injection device according to claim 38, wherein the syringe comprises a medication container and a pushrod coupled to the medication container, a needle with its proximal end connected to the medication container, the distal end of the pushrod being housed within the medication container, the proximal end of the pushrod pushes the distal end portion of the pushrod within the medication container upon being applied a force to deliver at least a portion of the substance in the medication container via the needle; the sleeve being provided with a canal and is movably coupled to the proximal portion of the needle, the distal end of the needle being configured to pierce ocular tissue through the sleeve canal via the gripping port.

40. The ocular injection device according to claim 39, wherein the ocular injection device performs injections with the sleeve forming a bottom-sealed chamber with the clamped ocular tissue via the clamping port.

41. The ocular injection device according to claim 39, wherein the clamping port has a shape of a circle, a hexagon, an octagon, a square or an irregular shape, preferably a circle; and the clamping port has a minimum inner diameter of 0.5 mm-10 mm, preferably 1 mm-6 mm, more preferably 1-3 mm.

42. The ocular injection device according to claim 39, wherein the length of the blade at the distal end of the needle is less than 1100 microns, preferably less than 900 microns, further preferably less than 700 microns, more preferably ≤ 550 microns, most preferably 250-550 microns; and the penetration force of the distal end of the needle is ≤ 0.7N, more preferably ≤ 0.5N.

43. The ocular injection device according to claim 39, wherein the ocular injection device applies a force on the ocular tissue capable of inducing an elastic deformation of the ocular tissue, the force being preferably 0.4N-10N, more preferably 2N-6N, and most preferably 3N-5N.

44. The ocular injection device according to claim 39, further comprising a needle hub coupled proximally to a distal end of a medication container and distally to a proximal end of the needle, with an adjustment assembly between the proximal end of the needle hub and the distal end of the needle hub cooperating with the sleeve in adjusting the length of the needle exposing the clamping port at the distal end of the sleeve.

45. The ocular injection device according to claim 39, wherein the ocular injection device comprises a needle hub coupled proximally to the distal end of a medication container and coupled distally to the proximal end of said the needle, the needle hub being provided with an adjustment release member between the needle hub and the sleeve configured to adjust the length of the needle exposing the distal end of the sleeve outside the clamping port and configured to be used by the ocular injection device to push the needle through the distal clamping port to pierce into the ocular tissue when the device performs a puncturing action.

46. The ocular injection device according to claim 39, wherein the injection device comprises a thrust assembly configured to generate a constant thrust force on the proximal end portion of the pushrod; and further preferably comprises a second stopper configured to selectively limit the movement of the pushrod with respect to the container of medication, the second stopper being configured to release the thrust assembly when performing an injection of the medication.

47. The ocular injection device according to any one of claims 38-46, wherein the sleeve is a transparent sleeve or is provided with a viewable window configured to facilitate observing the length of the needle exposed outside the sleeve clamping port, or \and configured to facilitate observing the amount of medicament return flow to the chamber.
